(19) Europäisches Patentamt
European Patent Office
Office européen des brevets

(11) EP 4 039 223 A1

(12) EUROPÄISCHE PATENTANMELDUNG

(43) Veröffentlichungstag:
**10.08.2022 Patentblatt 2022/32**

(21) Anmeldenummer: **22160681.7**

(22) Anmeldetag: **10.03.2020**

(51) Internationale Patentklassifikation (IPC):
***A61C 13/00** (2006.01)* ***A61K 6/62** (2020.01)*
***A61K 6/887** (2020.01)* ***B33Y 70/00** (2020.01)*
***B33Y 80/00** (2015.01)* ***A61C 7/08** (2006.01)*
***A61C 13/01** (2006.01)* ***A61C 13/34** (2006.01)*

(52) Gemeinsame Patentklassifikation (CPC):
(C-Sets verfügbar)
**A61K 6/62; A61C 7/08; A61C 13/0022;**
**A61C 13/04; A61C 13/34; A61K 6/887;**
**B33Y 70/00; B33Y 80/00;** A61C 2201/00 (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **11.03.2019 DE 102019106152**

(62) Dokumentnummer(n) der früheren Anmeldung(en) nach Art. 76 EPÜ:
**20712238.3 / 3 923 853**

(71) Anmelder: **Kulzer GmbH**
**63450 Hanau (DE)**

(72) Erfinder: **HOHMANN, Alfred**
**61389 Schmitten (DE)**

(74) Vertreter: **Bendele, Tanja**
**RUHR-IP Patentanwälte**
**Brucker Holt 58**
**45133 Essen (DE)**

Bemerkungen:
Diese Anmeldung ist am 08.03.2022 als Teilanmeldung zu der unter INID-Code 62 erwähnten Anmeldung eingereicht worden.

# (54) STRAHLENHÄRTBARE ZUSAMMENSETZUNG ZUR VERWENDUNG IN RAPID-PROTOTYPING- ODER RAPID-MANUFACTURING-VERFAHREN

(57) Gegenstand der Erfindung ist eine polymerisierbare, strahlenhärtbare Zusammensetzung umfassend
(i) Monomere, die umfassen mindestens (a) ein erstes Monomer mit einem TG (Glas übergangspunkt) des Homopolymers dieses ersten Monomers von größer gleich 120 °C und mindestens (b) ein zweites Monomer mit einem TG (Glasübergangspunkt) des Homopolymers des zweiten Monomers von kleiner gleich 100 °C, wobei mindestens eines des mindestens einen ersten Monomers oder des mindestens einen zweiten Monomers eine alicyclische Gruppe aufweist, und
umfassend (ii) mindestens eine weitere Komponente, die umfasst mindestens einen Photoinitiator für den UV- und/oder den Vis-Bereich oder ein Photoinitiatorsystem für den UV- und/oder den Vis-Bereich. Des Weiteren ist Gegenstand der Erfindung ein Rohling in Form eines dreidimensionalen Formkörpers einer polymerisierten Zusammensetzung, insbesondere einer strahlengehärteten Zusammensetzung zur Herstellung von 3D-Formkörper, wie dentaler Modelle, dentaler prothetischer Teile, orthopädischer Apparate oder dentaler Pre-Forms als auch die Verwendung der Zusammensetzung zur Herstellung dentaler prothetischer Teile, orthopädischer Apparate oder dentaler Pre-Forms in einem Rapid-Prototyping- oder in einem Rapid-Manufacturing- oder Rapid-Tooling-Verfahren.

EP 4 039 223 A1

(52) Gemeinsame Patentklassifikation (CPC): (Forts.)

C-Sets
**A61K 6/887, C08L 33/08;**
**A61K 6/887, C08L 33/10**

## Beschreibung

Gegenstand der Erfindung ist eine polymerisierbare, strahlenhärtbare Zusammensetzung umfassend

(i) Monomere, die umfassen mindestens (a) ein erstes Monomer mit einem TG (Glasübergangspunkt) des Homopolymers dieses ersten Monomers von größer gleich 120 °C und mindestens (b) ein zweites Monomer mit einen TG (Glasübergangspunkt) des Homopolymers des zweiten Monomers von kleiner gleich 100 °C, wobei mindestens eines des mindestens einen ersten Monomers oder des mindestens einen zweiten Monomers eine alicyclische Gruppe, insbesondere eine bicyclische Gruppe, aufweist, und optional mindestens ein difunktionelles Urethan-(meth)acrylat ausgewählt aus difunktionellen Urethan(meth)acrylaten mit bivalenter Alkylen-Gruppe, und umfassend (ii) mindestens eine weitere Komponente, die umfasst mindestens einen Photoinitiator für den UV und/oder den Vis-Bereich oder ein Photoinitiatorsystem für den UV und/oder den Vis-Bereich. Des Weiteren ist Gegenstand der Erfindung ein Rohling in Form eines dreidimensionalen Formkörpers einer polymerisierten Zusammensetzung, insbesondere einer strahlengehärteten Zusammensetzung zur Herstellung von 3D-Formkörpern, wie dentaler Modelle, dentaler prothetischer Teile, orthopädischer Apparate oder dentaler Pre-Forms als auch die Verwendung der Zusammensetzung zur Herstellung dentaler prothetischer Teile, orthopädischer Apparate oder dentaler Pre-Forms in einem Rapid-Prototyping- oder in einem Rapid-Manufacturing- oder Rapid-Tooling-Verfahren.

Neben handwerklichen Fertigungsmethoden gewinnen digitale Fertigungsmethoden wie subtraktive und additive Verfahren (Material-aufbauende Verfahren) im Dentalbereich immer mehr an Bedeutung. Der Vorteil bei additiven Verfahren liegt in der Einsparung von teuren Rohstoffen sowie einer schnelleren Fertigung der Objekte. Generative Verfahren sind im Dentalbereich z.B. schon in Form des Lasersinterns aus CoCr, Ti oder Polymeren zur Herstellung von Kronen und Brücken, Implantatbauteilen oder Modellen bekannt.

Zusammensetzungen von Acrylaten oder Derivaten von Acrylaten zur Herstellung von Zahnersatz mit entsprechendem Eigenschaftsprofil in Bezug auf die mechanischen Anforderungen im Dentalbereich nach der DIN EN ISO 20795-2 sind bislang nicht erhältlich (siehe Quintessenz Zahntechnik 2017-43(10): Seite 1325). Daher besteht grundsätzlich ein Bedarf an Materialien für die Herstellung von anatomischen Modellen, anatomischen Tischmodellen und insbesondere zur Herstellung anatomischer Modelle als Ersatz des dentalen Gipsmodells, das aus einer Abformung der Zahnsituation und der Gingiva bzw. des Gebisses eines Patienten hergestellt wird, oder zur Herstellung prothetischer Teile oder definitivem Zahnersatz. Ferner besteht somit auch ein Bedarf an Zusammensetzungen zur Herstellung von definitiven prothetischen Teilen, orthopädischen Apparaten oder dentalen Pre-Forms.

Die Abformung und das Modell, hier auch anatomisches Modell oder Arbeitsmodell genannt, bilden die Basis für passgenauen Zahnersatz. Nur wenn Zahnarzt und Zahntechniker die materialspezifischen Anforderungen bei Abformung und Modellherstellung einhalten, erhalten sie eine individuelle optimale Versorgung.

Zur Modellherstellung verwendet der Zahntechniker in der Regel Gips. Er ist einfach anzuwenden und erfüllt die Anforderungen an präzise, dimensionsgetreue, Oberflächen glatte Modelle. Nachfolgend werden die Anforderungen an ein dentales, anatomisches Gipsmodell wiedergegeben: Volumenstabilität, geringste Expansion, keine Kontraktion, Lagerungsstabilität, kompatibel mit Desinfektionsmitteln, Isoliermitteln und Wachsen, eine glatte und porenfreie Oberfläche, ausreichende Druckfestigkeit, hohe Kantenstabilität, gute Abrasionsbeständigkeit, hohe thermische Belastbarkeit, insbesondere beim Ausbrühen von Wachs, in Verbindung mit und ohne Wasser.

Aufgabe der vorliegenden Erfindung war es, eine Zusammensetzung bereitzustellen, die gute strahlenhärtbare Eigenschaften, insbesondere UV- und/oder Vis-Strahlen härtbare Eigenschaften, mit guter Polymerisationstiefe bei der Strahlenhärtung aufweist. Des Weiteren sollten die strahlengehärteten Zusammensetzungen sowohl bei Raumtemperatur als auch bei erhöhter Temperatur gute mechanische Eigenschaften aufweisen, um diese zur Herstellung von anatomischen Modellen, insbesondere dentalen Modellen, einsetzen zu können. Die Zusammensetzungen sollen daher die Anforderungen von Modell-Materialen, zur Herstellung von Modellen für Modellguss, Implantologie, Sägeschnitt- und Meistermodellen sowie präzisen Gegenbissmodellen erfüllen. Es bestand daher auch die Aufgabe lichthärtende, in additiven/ generativen Verfahren verwendbare Zusammensetzungen bereitzustellen, die als gedruckter 3D-Formkörper im gehärteten Zustand die folgenden Anforderungen erfüllen: Beibehaltung der mechanischen Eigenschaften und Dimensionsstabiliät bei Temperaturen von 45 °C bis 55 °C im Drucktopf, Dimensionsstabiliät im Tiefziehprozess, sowie auch bei einer Reinigung bspw. mit einem Dampfstrahler, Lagerstabilität der lichthärtenden Zusammensetzung ohne signifikante Viskositätsänderung über den Lagerzeitraum, ausreichende Reaktivität bei Belichtung mit Laser, LED oder DLP-Projektor, Werkstücke/Formkörper mit ausreichender geometrischer Präzision/Auflösung druckbar, Farbstabilität der Mischung, und keine oder nur geringe Thixotropie. Aufgabe der Erfindung war es daher eine strahlenhärtbare Zusammensetzung bereitzustellen, die polymerisiert bei einer Prüfung in Wasser bei 55 °C eine Biegefestigkeit von mindestens 40 MPa und ein Biegemodul synonym zu E-Modul von mindestens 800 MPa aufweist.

Nachfolgend sind die weiteren Anforderungen an ein dentales Gipsmodell in verschiedenen Arbeitsschritten skizziert: Modell in Verbindung mit thermischen Einflüssen und Wasser: Angießen von Kunststoffsattel bei Modellguss

(20 Min. bei 55 °C im Wasserbad), Fertigstellung einer Teilprothese/Modellguss (20-30 Min. bei 55 °C im Wasserbad), Fertigstellung einer Totalprothese (Injektionstechnik Palajet/Küvetten Technik - 30 Min. bei 55 °C im Wasserbad), Fertigstellung einer Totalprothese Stopf-Presstechnik (30-40 Min. bei 100 °C im Wasserbad), Tiefziehschienen (20 - 30 Min. bei 55 °C), Wasserdampf zur Reinigung (70 °C - 110 °C), Abbrühen (3-5 Min. bei 80 °C - 100 °C), Verblendung mit Pala Veneers (55 °C, 2 bar, 20 Min.).

Modell in Verbindung mit Isolierungen und mechanischen Einflüssen: Ein-Artikulieren, d.h. das Modell wird im Artikulator mechanisch belastet, Isolierung gegen Prothesenkunststoff, Isolierung gegen Verblendkomposit, Isolierung gegen Wachs, Ausblockbarkeit mit UV-Kunststoff und/oder Wachs, Tauchwachs.

Modell in Verbindung mit thermischen Einflüssen ohne Wasser: Tiefziehen (155 - 170 °C/1 bis 2 Minuten), Heißklebepistole zum Fixieren der Modelle, Klebewachs zum Fixieren der Modelle, Ausblockbarkeit mit UV-Kunststoff und/oder Wachs, Tauchwachs, Tiefziehfolie und manuelle Härtung von Kompositen in Hi Lite Power/3D (zweimal je 90 Sek. und einmal 180 Sek.), Härtung von Aufbissschienen in Hi Lite Power/3D (zweimal je 5 Min.), Frässtumpf für Frästechnik (temperaturbeständig gegen Stauhitze).

Verhalten der polymerisierten Zusammensetzung bei anderen Arbeitsschritten: Dimensionsstabil und gute Reinigungseigenschaften im Ultraschallbad mit Isopropanol, Verfärbung und Reinigung bei Artikulationsfolie (Bausch, rot, blau, schwarz), Verfärbung und Reinigung bei Okklusionsspray (verschiedene Hersteller), Abriebfestigkeit an der Präparationsgrenze (Kronen, Brücken, Modellguss-Klammerzähne), sprung/abriebfest (im Artikulator - Gips gegen Kunststoff; Kunststoff gegen Kunststoff).

Situationsmodelle sowie alle Arbeitsmodelle stellen die Grundlage für alle weiteren zahntechnischen Arbeiten dar. Zur Erfüllung hoher ästhetischer Ansprüche müssen Harz-Mischungen für den definitiven Zahnersatz, wie z.B. Arbeitsmodelle, KFO-Modelle Dimensionsstabilität und Thermostabilität aufweisen. Möchte man Gipsmodelle durch gedruckte Kunststoffmodelle ersetzen um die Vorteile des digitalen Workflows zu erhalten, müssen die Kunststoffmodelle die gleichen positiven Eigenschaften wie Gipse aufweisen. Aufgrund verschiedener physikalischer und chemischer Randbedingungen unterliegen Kunststoffe aber einer thermischen Plastizität, die es gilt durch eine optimale Auswahl der Monomere in Grenzen zu halten.

Bisher gibt es keine Materialien, die alle genannten thermostabilen Eigenschaften aufweisen, so dass diese nur für einige zahntechnische Anwendungen verwendbar sind.

Der Erfindung lag daher ebenfalls die Aufgabe zu Grunde eine Zusammensetzung umfassend Monomere bereitzustellen, die eine besonders hohe Thermostabilität und Dimensionstreue aufweist, um bei Verwendung der polymerisierten Zusammensetzung als Formteil im Drucktopf sowie beim Tiefziehen von Aufbissschienen den erhöhten Temperaturen standzuhalten. Bei der Herstellung von Alignern muss mehrfach den höheren Temperaturen standgehalten werden.

Gelöst werden die Aufgaben der Erfindung durch die Zusammensetzung nach Anspruch 1 sowie die polymerisierte Zusammensetzung nach Anspruch 11 als auch den Rohling nach Anspruch 13 und die Verwendung nach Anspruch 14. Bevorzugte Ausführungsformen werden in den Unteransprüchen und detailliert in der Beschreibung offenbart.

Gegenstand der Erfindung sind polymerisierbare, strahlenhärtbare Zusammensetzungen umfassend (i) Monomere, wobei die Monomere mindestens (a) ein erstes Monomer mit einem TG (Glasübergangspunkt) des Homopolymers dieses ersten Monomers von größer gleich 120 °C umfassen und mindestens (b) ein zweites Monomer mit einem TG (Glasübergangspunkt) des Homopolymers des zweiten Monomers von kleiner gleich 100 °C umfassen, wobei mindestens eines des mindestens einen ersten Monomers oder des mindestens einen zweiten Monomers eine alicyclische Gruppe, insbesondere eine mindestens bicyclische alicyclische Gruppe, aufweist, und wobei in der Zusammensetzung vorliegen

(i) 40 bis 99,99 Gew.-% (a) mindestens ein erstes Monomer oder eine Mischung von ersten Monomeren mit einem TG (Glasübergangspunkt) des jeweiligen Homopolymers des entsprechenden ersten Monomers von größer gleich 120 °C vorliegen und umfasst mindestens ein difunktionelles Acrylat mit bivalenter alicyclischer Gruppe und/oder difunktionelles Methacrylat mit bivalenter alicyclischer Gruppe und mindestens ein difunktionelles Urethan(meth)acrylat ausgewählt aus difunktionellen Urethan(meth)acrylaten mit bivalenter Alkylen-Gruppe, und

- bis 60 Gew.-% mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C vorliegen, und die zweiten Monomere umfassen mindestens ein monofunktionelles Acrylat mit alicyclischer Gruppe und/oder monofunktionelles Methacrylat mit alicyclischer Gruppe,

(ii) 0,01 bis 5 Gew.-% mindestens einer weiteren Komponente, umfassend 0,01 bis 5 Gew.-% der mindestens einen weiteren Komponente vorliegen, die mindestens einen Photoinitiator für den UV- und/oder den Vis-Bereich oder ein Photoinitiatorsystem für den UV- und/oder den Vis-Bereich und optional mindestens einen Stabilisator für den

UV- und/oder Vis-Bereich und optional mindestens ein Pigment und/oder Farbstoff sowie weitere übliche Additive, wobei die Gesamtzusammensetzung 100 Gew.-% beträgt und, insbesondere wobei die Viskosität der Zusammensetzung bei Raumtemperatur (ca. 20 °C bis 23 °C) kleiner gleich 3000 m·Pas, insbesondere von 500 bis kleiner 2500 m·Pas ist.

Unter dem Begriff Monomere werden auch Mischungen der genannten Monomere, wie u.a. Mischungen umfassend erste Monomere und zweite Monomere verstanden. Unter ersten Monomeren werden (i) ein erstes Monomer als auch (ii) Mischungen von ersten Monomeren und unter zweiten Monomeren (i) ein zweites Monomer als auch (ii) Mischungen von zweiten Monomeren verstanden. Der TG wird vorzugsweise mittels DSC bestimmt.

Besonders bevorzugte Zusammensetzungen weisen eine Viskosität bei Raumtemperatur auf, die geeignet ist für die Verwendung dieser in generativen strahlenhärtenden Verfahren. Daher weisen die Zusammensetzungen vorzugsweise eine Viskosität von kleiner 5000 m·Pas auf. Besonders bevorzugt ist die Viskosität der Zusammensetzung bei Raumtemperatur (ca. 20 °C bis 23 °C) kleiner gleich 3000 m·Pas, insbesondere von 500 bis kleiner 2500 m·Pas.

Nach einer bevorzugten Ausführungsform weisen die Zusammensetzungen eine Mischung von ersten und zweiten Monomeren auf, wobei die ersten Monomere umfassen difunktionelle Acrylate mit bivalenter alicyclischer Gruppe und difunktionelle Methacrylate mit bivalenter alicyclischer Gruppe, insbesondere bivalente bicyclische Acrylate oder bivalente bicyclische Methacrylate, bevorzugt jeweils von bivalenten Tetrahydrodicyclopentadienen, und die zweiten Monomere umfassen mindestens ein monofunktionelles Acrylat mit alicyclischer Gruppe und/oder monofunktionelles Methacrylat mit alicyclischer Gruppe, besonders bevorzugt ist eine Mischung von monofunktionellem Acrylat mit bicyclischer alicyclischer Gruppe und einem difunktionellen Acrylat mit bicyclischer alicyclischer Gruppe. Gleichfalls bevorzugt sind die jeweiligen Methacrylate dieser Monomere.

Nach einer alternativen bevorzugten Ausführungsform ist eine Zusammensetzung bevorzugt, die als (i) Monomere umfasst

(a) insbesondere als mindestens ein erstes Monomer, mindestens ein aus 1,3,5-Tris(hydroxyalkyl)isocyanurat abgeleitetes Triacrylat, wobei der Hydroxyalkyl-Rest jeweils unabhängig 1 bis 8 C-Atome umfasst und insbesondere mit 3 bis 8 C-Atomen linear, verzweigt und/oder cyclisch ist, insbesondere 1 bis 6 C-Atome, bevorzugt 1 bis 4 C-Atome, bevorzugt 1 bis 3 C-Atome, besonders bevorzugt ist Hydroxyethyl, und optional

(b) difunktionelle Acrylate mit bivalenter alicyclischer Gruppe und/oder difunktionelle Methacrylate mit bivalenter alicyclischer Gruppe, und optional

(c) insbesondere als mindestens ein zweites Monomer, mindestens ein monofunktionelles Acrylat mit alicyclischer Gruppe und/oder monofunktionelles Methacrylat mit alicyclischer Gruppe.

Optional kann (d) als ein Monomer, vorzugsweise als mindestens ein drittes Monomer, mindestens ein disubstituiertes 4,4'-Di(oxabenzol)dialkylmethan der Formel I in der Zusammensetzung vorliegen,

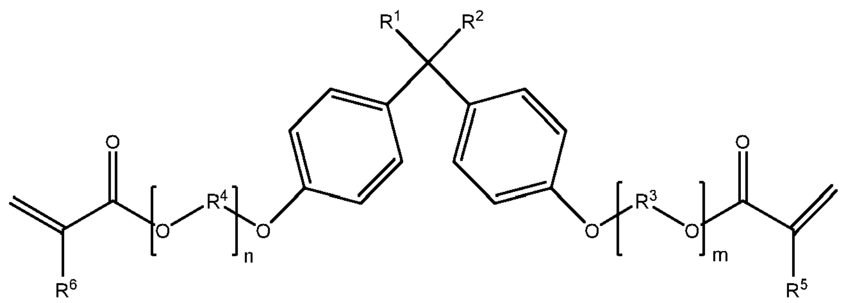

(I)

mit $R^1$, $R^2$, $R^5$ und $R^6$ jeweils unabhängig ausgewählt aus H oder C1- bis C4-Alkyl, und mit $R^3$ und $R^4$ jeweils bivalent C1- bis C4-Alkylen, mit n = 0 bis 6 und m = 0 bis 6. Je nach Substitutionsmuster ist das disubstituierte 4,4'-Di(oxabenzol)dialkylmethan der Formel I ein erstes oder ein drittes Monomer. Mit n und m = 2 und $R^1$, $R^2$, $R^5$ und $R^6$ gleich Methyl und $R^4$ und $R^3$ gleich Ethylen ist es ein drittes Monomer. Mit Formel I gleich n = 2 und m = 2 und $R^1$, $R^2$, $R^5$ und $R^6$ gleich Methyl und $R^4$ und $R^3$ gleich Ethylen ist das Monomer ein drittes Monomer, das mit 0 bis 50 Gew.-% in der Gesamtzusammensetzung vorliegen kann.

Dabei kann das Monomer (d) ein erstes oder ein drittes Monomer sein, je nachdem, wie die Reste $R^1$ bis $R^6$ und die Indices n und m gewählt werden, kann der TG größer gleich 120 °C betragen, so dass es ein erstes Monomer ist. Das dritte Monomer weist einen TG größer gleich 100 °C und vorzugsweise kleiner 120 °C auf, und wird vorzugsweise nur von 0 bis kleiner 40 Gew.-% in der Gesamtzusammensetzung eingesetzt. Mit einem TG größer gleich 30 °C und kleiner 50 °C, liegt das Monomer von 0 bis kleiner 10 Gew.-% in der Gesamtzusammensetzung vor.

Vorzugsweise weist die Zusammensetzung nur von 0 bis 7,5 Gew.-%, bevorzugt keine signifikanten Anteile, vorzugsweise lediglich 0 bis 0,05 Gew.-%, mindestens eines Acrylsäureesters mit zusätzlicher Carboxy-Gruppe auf, wie mindestens einen Acrylsäureester mit mindestens einer zusätzlichen Anhydrid-Gruppe von Carboxy-Gruppen und/oder mindestens ein Derivat der vorgenannten Acrylsäureester.

Eine weitere bevorzugte Ausführungsform einer Zusammensetzung umfasst (i) Monomere, die (a) mindestens ein erstes Monomer oder eine Mischung von ersten Monomeren mit einem TG (Glasübergangspunkt) des Homopolymers des jeweiligen ersten Monomers von größer gleich 120 °C umfassen, wobei das erste Monomer oder die Mischung von ersten Monomeren mindestens einen Diacrylatester basierend auf einem Tricyclodecan-dialkanol, mit Alkanol umfassend 1 bis 6 C-Atome, oder eine Mischung dieser umfasst, und (b) mindestens ein zweites Monomer oder eine Mischung von zweiten Monomeren mit jeweils einem TG (Glasübergangspunkt) des Homopolymers des jeweiligen zweiten Monomers von kleiner gleich 100 °C umfassen, wobei das zweite Monomer oder die Mischung von zweiten Monomeren mindestens einen mono-funktionellen Acrylatester basierend auf einem Tricyclodecanalkanol (synonym zu (Octahydro-4,7-methano-1H-indenyl)alkanol), mit Alkanol umfassend 1 bis 6 C-Atome, oder eine Mischung dieser und optional mindestens ein difunktionelles Urethan(meth)acrylat ausgewählt aus difunktionellen Urethan(meth)acrylaten mit bivalenter Alkylen-Gruppe, umfasst, wobei optional

(i) in der Zusammensetzung 15 bis 25 Gew.-% mono-funktioneller Acrylatester basierend auf einem Tricyclodecanalkanol, mit Alkanol umfassend 1 bis 6 C-Atome, und optional 5 bis 15 Gew.-% Diacrylatester basierend auf einem Tricyclodecan-dialkanol, mit Alkanol umfassend 1 bis 6 C-Atome, vorliegen, wobei die Gesamtzusammensetzung 100 Gew.-% beträgt, oder
(ii) 5 bis 15 Gew.-% Diacrylatester basierend auf einem Tricyclodecan-dialkanol, mit Alkanol umfassend 1 bis 6 C-Atome, und 15 bis 25 Gew.-% mono-funktioneller Acrylatester basierend auf einem Tricyclodecanalkanol, mit Alkanol umfassend 1 bis 6 C-Atome, vorliegen, insbesondere in (ii) ohne die Gegenwart von aus 1,3,5-Tris(hydroxyalkyl)isocyanurat abgeleitetem Triacrylat, wobei die Gesamtzusammensetzung 100 Gew.-% beträgt, oder
(iii) 15 bis 45 Gew.-% Mischung umfassend mono-funktionellen Acrylatester basierend auf einem Tricyclodecanalkanol, mit Alkanol umfassend 1 bis 6 C-Atome, und Diacrylatester basierend auf einem Tricyclodecan-dialkanol, mit Alkanol umfassend 1 bis 6 C-Atome, insbesondere in (iii) ohne die Gegenwart von aus 1,3,5-Tris(hydroxyalkyl)isocyanurat abgeleitetem Triacrylat, wobei die Gesamtzusammensetzung 100 Gew.-% beträgt.

Ferner kann die Zusammensetzung vorzugsweise umfassen, als mindestens erste Monomere (a.1) das mindestens eine aus 1,3,5-Tris(hydroxyalkyl)isocyanurat abgeleitete Triacrylat umfassend 1,3,5-Tris(2-hydroxyethyl)isocyanurat-Triacrylat, 1,3,5-Tris(2-hydroxymethyl)-isocyanurat-Triacrylat, 1,3,5-Tris(2-hydroxyethyl)isocyanurat-Trimethacrylat, 1,3,5-Tris(2-hydroxymethyl)isocyanurat-Trimethacrylat oder Mischungen enthaltend mindestens zwei der Monomere.

Bevorzugte erste Monomere umfassen (a) und sind ausgewählt aus Tricyclodecandimethanoldiacrylat, Tricyclodecandimethanoldimethacrylat, Tricyclodecandiethanoldiacrylat, Tricyclodecandiethanoldimethacrylat und/oder Mischungen dieser. Die vorstehenden Tricyclodecan-Monomere können alle Strukturisomere der Monomere umfassen.

Alternative Monomere, insbesondere dritte Monomere, von 0 bis kleiner gleich 10 Gew.-%, umfassen Monomere mit einem TG größer gleich 100 °C und vorzugsweise kleiner 120 °C. Daher kann die Zusammensetzung ferner Monomere umfassen wie (d) mindestens ein disubstituiertes 4,4'-Di(oxabenzol)dialkylmethan der Formel I mit $R^1$, $R^2$, $R^5$ und $R^6$ jeweils unabhängig ausgewählt aus H oder C1-Alkyl, und mit $R^3$ und $R^4$ jeweils bivalent C1- bis C3-Alkylen, insbesondere C2-Alkylen, mit n = 1 bis 6 und m = 1 bis 6. Alternativ bevorzugt sind $R^1$ und $R^2$ jeweils Methyl, und $R^5$ und $R^6$ gleich und ausgewählt aus H, Methyl und Ethyl, insbesondere mit $R^5$ und $R^6$ gleich und ausgewählt aus H und Methyl, und mit $R^3$ und $R^4$ jeweils unabhängig bivalent Ethylen oder Propylen mit n = 1 bis 6, bevorzugt n = 2 bis 4, und mit m = 1 bis 6, bevorzugt m = 2 bis 4, bevorzugt mit n = 2 und m = 2 oder mit n = 4 und m = 4 sowie Mischungen dieser. Je nachdem wie hoch der jeweilige TG des spezifisch substituierten disubstituierten 4,4'-Di(oxabenzol)dialkylmethans der Formel I ist, kann dieses Monomer entweder ein erstes oder zweites oder drittes Monomer sein. Vorzugsweise ist die Zusammensetzung frei von Monomeren der Formel I, insbesondere mit n und m größer gleich 4 oder enthält diese von 0 bis kleiner gleich 7,5 Gew.-% in Bezug auf die Gesamtzusammensetzung.

Nachfolgend sind einige Monomere der Formel I angegeben, teilweise mit ihrem TG: ethoxyliertes(2)Bisphenol ADimethacrylat (TG 105°C), ethoxyliertes(2)Bisphenol A Diacrylat, ethoxyliertes(3)Bisphenol A Dimethacrylat (TG 115°C), ethoxyliertes(3)Bisphenol A Diacrylat (TG 67 °C), ethoxyliertes(4)Bisphenol A Dimethacrylat (TG 90 °C), ethoxyliertes(4)Bisphenol A Diacrylat (TG 60°C), ethoxylierstes (10)Bisphenol A Dimethacrylat (TG (-1 °C)), ethoxyliertes (10)Bisphenol A Diacrylat ( TG 2 °C).

Nach einer Alternative kann das zweite Monomer oder die Mischung von zweiten Monomeren umfassen mindestens ein Monomer oder eine Mischung von zweiten Monomeren mit einem TG des jeweiligen Homopolymers von größer gleich 30 °C, bevorzugt umfasst sind zweite Monomere mit einem TG im Bereich von größer gleich 30 °C bis kleiner gleich 100 °C, besonders bevorzugt sind zweite Monomere mit einem TG im Bereich von größer gleich 50 °C

bis kleiner gleich 100 °C. Weitere bevorzugte Zusammensetzungen umfassen (i) Monomere umfassend erste Monomere, zweite Monomere und dritte Monomere sowie die zusätzliche Komponente, die vorzugsweise mindestens einen UV-, Vis- oder UV/Vis-Stabilisator umfasst.

Nach einer Alternative sind Zusammensetzungen umfasst, in denen die (i) Monomere

- 40 bis 99,99 Gew.-% des (a) mindestens einen ersten Monomers oder einer Mischung von ersten Monomeren mit einem TG (Glasübergangspunkt) des jeweiligen Homopolymers des entsprechenden ersten Monomers von größer gleich 120 °C vorliegt, und
- 0 bis 60 Gew.-%, insbesondere 0 bis 10 Gew.-%, des (b) mindestens einen zweiten Monomers oder einer Mischung von zweiten Monomeren mit einem TG (Glasübergangspunkt) des jeweiligen Homopolymers des entsprechenden zweiten Monomers von kleiner gleich 100 °C vorliegt,
- 0,01 bis 5 Gew.-% der mindestens einen weiteren Komponente umfassend mindestens einen Photoinitiator für den UV- und/oder den Vis-Bereich oder ein Photoinitiatorsystem für den UV- und/oder den Vis-Bereich und optional mindestens einen Stabilisator für den UV- und/oder Vis-Bereich und optional mindestens ein Pigment und/oder Farbstoff sowie weitere übliche Additive,

wobei die Gesamtzusammensetzung 100 Gew.-% beträgt und, insbesondere wobei die Viskosität der Zusammensetzung bei Raumtemperatur (ca. 20 °C bis 23 °C) kleiner gleich 3000 m·Pas, insbesondere von 500 bis kleiner 2500 m·Pas ist.

In weiteren alternativen Ausführungsformen kann eine Zusammensetzung als

(i) das erste Monomer umfassen
(e) mindestens ein difunktionelles Urethan(meth)acrylat, das ausgewählt ist aus difunktionellen Urethan(meth)acrylaten mit bivalenter Alkylen-Gruppe, und/oder
(f.1) mindestens ein mono-, tri-, tetra- oder multi-funktionelles Monomer, das insbesondere kein Urethan(meth)acrylat ist, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyether

Bevorzugt sind Zusammensetzungen, die umfassen:

(i) das zweite Monomer umfassend
(c) mindestens ein monofunktionelles Acrylat mit alicyclischer Gruppe und/oder mono-funktionelles Methacrylat mit alicyclischer Gruppe, insbesondere ausgewählt aus (Octahydro-4,7-methano-1H-indenyl)methanolacrylat, (Octahydro-4,7-methano-1H-indenyl)methanolmethacrylat, (Octahydro-4,7-methano-1H-indenyl)ethanolacrylat und (Octahydro-4,7-methano-1H-indenyl)ethanolmethacrylat,
(f.2) mindestens ein mono-, di- tri-, tetra- oder multi-funktionelles Monomer, das insbesondere kein Urethan(meth)acrylat ist, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern.

Geeignet sind auch Trimethylolpropan-Triacrylat (TG 60 °C), Di-Pentaerythritol-Pentaacrylat (TG 90 °C), ethoxyliertes(4)Pentaerythritol-Tetraacrylat (TG 70 °C) und ethoxyliertes (4)Pentaerythritol-Tetraacrylat (TG 70 °C). Trifunktionelle Monomere können zudem ausgewählt sein aus, wobei diese vorzugsweise nur mit 0 bis 10 Gew.-%, insbesondere mit 0,01 bis 5 Gew.-% in der Gesamtzusammensetzung eingesetzt werden: Ethoxyliertes (20)Trimethylolpropan-Triacrylat (TG -40 °C), ethoxyliertes(3)Trimethylolpropan-Triacrylat, Propoxyliertes (3)Triemethylolpropan-Triacrylat, ethoxyliertes(6)Trimethylolpropan-Triacrylat (TG -10 °C), ethoxyliertes(9)Trimethylolpropan-Triacrylat (TG -20 °C), propoxyliertes (3)Glyceryl-Triacrylat (TG 20 °C), ethoxyliertes(15)Trimethylolpropan-Triacrylat (TG -30 °C). Dabei soll die Viskosität der Zusammensetzug kleiner 3000 m·Pas liegen.

Gleichfalls bevorzugte Zusammensetzungen können zusätzlich zum ersten Monomer auch (iii) dritte Monomere, insbesondere von 0 bis 15 Gew.-%, optional von 1 bis 10 Gew.-%, umfassen, wobei die dritten Monomere umfassen können
(f.3) mindestens ein mono-, tri-, tetra- oder multi-funktionelles Monomer, das insbesondere kein Urethan(meth)acrylat ist, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern.

Bespiele für Tetraacrylate, die den dritten Monomeren zuzurechnen sind: Pentaerythritol-Tetraacrylat (TG 105 °C), Di-Trimethylolpropan-Tetraacrylat (TG 100 °C).

Nach einer weiteren alternativen Ausführungsform kann die Zusammensetzung umfassen (i) Monomere umfassend:

- 70 bis 99,99 Gew.-%, insbesondere 80 bis 99,99 Gew.-% (a) erstes Monomer oder Mischung von ersten Monomeren mit einem TG (Glasübergangspunkt) des jeweiligen Homopolymers des entsprechenden ersten Monomers von größer gleich 120 °C, wobei das erste Monomer oder die Mischung von ersten Monomeren umfasst mindestens ein difunktionelles Acrylat mit bivalenter alicyclischer Gruppe und/oder mindestens ein difunktionelles Methacrylat mit bivalenter alicyclischer Gruppe und optional mindestens ein difunktionelles Urethan(meth)acrylat ausgewählt aus difunktionellen Urethan(meth)acrylaten mit bivalenter Alkylen-Gruppe, und
- 0 bis 30 Gew.-%, insbesondere 0 bis 20 Gew.-%, bevorzugt 1 bis 15 Gew.-%, mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C, wobei das zweite Monomer oder die Mischung von zweiten Monomeren umfasst mindestens ein zweites Monomer oder eine Mischung von zweiten Monomeren mit einem TG des jeweiligen Homopolymers von größer gleich 30 °C, bevorzugt umfasst sind zweite Monomere mit einem TG im Bereich von größer gleich 30 °C bis kleiner gleich 100 °C, besonders bevorzugt sind zweite Monomere mit einem TG im Bereich von größer gleich 50 °C bis kleiner gleich 100 °C, insbesondere mono- und/oder difunktionelle Acrylate und/oder Methacrylate, bevorzugt von bicyclischen, alicyclischen Acrylaten und/oder Methacrylaten, und optional

- 0 bis 10 Gew.-% eines dritten Monomers mit einem TG des jeweiligen Homopolymers von größer 100 °C, insbesondere TG größer gleich 105 °C bis kleiner gleich 115 °C, insbesondere kleiner gleich 118 °C, und
- 0,01 bis 5 Gew.-% der mindestens einen weiteren Komponente umfassend mindestens einen Photoinitiator für den UV- und/oder den Vis-Bereich oder ein Photoinitiatorsystem für den UV und/oder den Vis-Bereich und optional mindestens einen Stabilisator für den UV- und/oder den Vis-Bereich und optional mindestens ein Pigment und/oder Farbstoff sowie weitere übliche Additive.

Gleichfalls kann eine Zusammensetzung umfassen

- 75 bis 99,99 Gew.-%, insbesondere 80 bis 99,99 Gew.-%, (i) (a) erstes Monomer oder Mischung von ersten Monomeren mit einem TG (Glasübergangspunkt) des jeweiligen Homopolymers des entsprechenden ersten Monomers oder der Mischung von ersten Monomeren von größer gleich 120 °C, wobei das erste Monomer oder die Mischung von ersten Monomeren umfasst mindestens ein difunktionelles Acrylat mit bivalenter alicyclischer Gruppe und/oder mindestens ein difunktionelles Methacrylat mit bivalenter alicyclischer Gruppe, und optional mindestens ein difunktionelles Urethan(meth)acrylat ausgewählt aus difunktionellen Urethan(meth)acrylaten mit bivalenter Alkylen-Gruppe, und
- 0 bis 25 Gew.-%, insbesondere 0 bis 20 Gew.-%, bevorzugt 1 bis 20 Gew.-%, mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C, wobei das zweite Monomer oder die Mischung von zweiten Monomeren umfasst mindestens ein monofunktionelles Acrylat mit alicyclischer Gruppe und/oder monofunktionelles Methacrylat mit alicyclischer Gruppe, mit
- 0,01 bis 5 Gew.-% der mindestens einen weiteren Komponente umfassend mindestens einen Photoinitiator für den UV- und/oder den Vis-Bereich oder ein Photoinitiatorsystem für den UV- und/oder den Vis-Bereich und optional mindestens einen Stabilisator für den UV- und/oder Vis-Bereich und optional mindestens ein Pigment und/oder Farbstoff sowie weitere übliche Additive, wobei die Gesamtzusammensetzung 100 Gew.-% beträgt, und, insbesondere wobei die Viskosität der Zusammensetzung bei Raumtemperatur (ca. 20 °C bis 23 °C) kleiner gleich 3000 m·Pas, insbesondere von 500 bis kleiner 2500 m·Pas ist.

In besonders bevorzugten Zusammensetzungen ist das erste Monomer zu 70 bis 99,99 Gew.-%, insbesondere 80 bis 99,99 Gew.-% in der Zusammensetzung enthalten und kann umfassen in Bezug auf die Gesamtzusammensetzung eine Mischung von ersten Monomeren umfassend 5 bis 50 Gew.-%, insbesondere 5 bis 25 Gew.-%, bevorzugt 5 bis 20 Gew.-% als mindestens ein difunktionelles Acrylat mit bivalenter alicyclischer Gruppe und/oder mindestens ein difunktionelles Methacrylat mit bivalenter alicyclischer Gruppe und optional 5 bis 50 Gew.-%, insbesondere 5 bis 45 Gew.-%, bevorzugt 7,5 bis 30 Gew.-% mindestens ein difunktionelles Urethan(meth)acrylat ausgewählt aus difunktionellen Urethan(meth)acrylaten mit bivalenter Alkylen-Gruppe. Optional können weitere erste Monomere in der Mischung von ersten Monomeren enthalten sein.

Eine weitere besonders bevorzugte Zusammensetzung umfasst (i) Monomere und die (ii) mindestens eine weitere Komponente, wobei ein TG(gesamt) ermittelt wird aus 1/TG(gesamt) der Monomere, wobei 1/TG(gesamt) nach folgender Formel ermittelt wird, mit

$$1/TG(gesamt) = w1/TG_{(1)} + w2/TG_{(2)} + w3/TG_{(3)} + \text{optional } w4/TG_{(4)} + \text{optional } w5/TG_{(5)} + \text{optional } wn/TG_{(n)},$$

mit w1, w2, w3, w4, w5 und wn jeweils Gewichtsanteil des jeweiligen Monomers, insbesondere des jeweiligen ersten Monomers, des jeweiligen zweiten Monomers oder optional des jeweiligen dritten Monomers, in der Gesamtmischung der mindestens ersten und zweiten Monomere von 100 Gew.-%, mit n = 6 bis 50, wobei TG(gesamt) größer gleich 80 °C, insbesondere größer gleich 100 °C, bevorzugt größer gleich 120 °C, vorzugsweise größer gleich 170 °C, ist, und die Viskosität der Zusammensetzung bei Raumtemperatur (ca. 20 °C bis 23 °C) kleiner gleich 3000 m·Pas, insbesondere von 500 bis kleiner 2500 m·Pas ist.

Dabei umfassen (i) die Monomere mindestens (a) ein erstes Monomer mit einem TG (Glasübergangspunkt) des Homopolymers dieses ersten Monomers von größer gleich 120 °C und mindestens (b) ein zweites Monomer mit einen TG (Glasübergangspunkt) des Homopolymers des zweiten Monomers von kleiner gleich 100 °C, wobei das zweite Monomer oder die Mischung von zweiten Monomeren umfasst mindestens ein zweites Monomer oder eine Mischung von zweiten Monomeren mit einem TG des jeweiligen Homopolymers von größer gleich 30 °C, bevorzugt umfasst sind zweite Monomere mit einem TG im Bereich von größer gleich 30 °C bis kleiner gleich 100 °C, besonders bevorzugt sind zweite Monomere mit einem TG im Bereich von größer gleich 50 °C bis kleiner gleich 100 °C, (ii) die mindestens eine weitere Komponente umfasst mindestens einen Photoinitiator für den UV- und/oder den Vis-Bereich oder ein Photoinitiatorsystem für den UV- und/oder Vis-Bereich.

Eine Ausführungsform umfasst eine polymerisierte Zusammensetzung, insbesondere erhältlich durch Strahlenhärtung der Zusammensetzung, wobei der TG(gesamt) über 1/TG(gesamt) nach der folgenden Formel ermittelt wird, und die (i) Monomere gemäß folgenden Gewichtsanteilen (w1, w2, w3, w4, w5 und wn) in der Gesamtzusammensetzung vorliegen.

$$1/TG(gesamt) = w1/TG_{(1)} + w2/TG_{(2)} + w3/TG_{(3)} + \text{optional } w4/TG_{(4)} + \text{optional } w5/TG_{(5)} + \text{optional } wn/TG_{(n)},$$

mit w1, w2, w3, w4, w5 und wn jeweils Gewichtsanteil des Monomers in der Gesamtzusammensetzung der ersten und zweiten Monomere und optional des dritten Monomers von 100 Gew.-%, mit n = 6 bis 50, wobei TG größer gleich 100 °C beträgt, und die polymerisierte Zusammensetzung eine Biegefestigkeit von größer gleich 40 MPa (angelehnt an DIN EN ISO 20795-2), insbesondere gemessen in Wasser bei 55 °C, aufweist und/oder b) ein E-Modul von größer gleich 800 MPa (angelehnt an DIN EN ISO 20795-2), insbesondere gemessen in Wasser bei 55 °C aufweist.

Die Glasübergangstemperatur von Co-Polymeren können angenähert über die Fox-Gleichung angegeben werden, siehe vorstehend und [Bullentin of the American Physical Society 1, 3 Page 123 (1956]).

Teilkristalline Kunststoffe (viele gebräuchliche Kunststoffe weisen einen kristallinen Anteil von 10 bis 80 % auf) besitzen sowohl eine Glasübergangstemperatur, unterhalb derer die amorphe Phase einfriert (einhergehend mit Versprödung), als auch eine Schmelztemperatur, bei der sich die kristalline Phase auflöst. Ein Glasübergang ist kein Phasenübergang 1. Ordnung und daher auch nicht mit einer exakten Temperatur verknüpft wie der Schmelzpunkt bei Kristallen. Je nachdem, auf welcher Zeit- und Längenskala bzw. welchem Bewegungsmodus der molekularen Dynamik die verwendete Messmethode (s. u.) empfindlich ist, variiert der gefundene Wert systematisch. Ob ein Kunststoff oberhalb oder unterhalb seiner Glasübergangstemperatur verwendet werden kann, hängt von der Art des Kunststoffs ab (dabei ist zu beachten, dass die Glasübergangstemperatur eines Kunststoffes mit seiner Vernetzungsdichte steigt, d. h. die Glasübergangstemperatur eines Duroplasts ist deutlich höher als die eines Thermoplasts).

$1/Tg = w1/Tg_{(1)} + w2/Tg_{(2)}$ mit w1 und w2 als Massenanteil der jeweiligen Co-Monomere und $Tg_{(1)}$ und $Tg_{(2)}$ für die jeweilige Glasübergangstemperatur der Homopolymere der Monomere 1 und 2. Bei weiteren Co-Monomeren werden weitere Terme (wn/Tg(n)) in die Gleichung eingefügt. Die offenbarten Glasübergangstemperaturen können den dem Fachmann bekannten "Polymer Handbüchern", den Angaben der Hersteller der Monomere entnommen werden. Soweit keine Angaben zu Glasübergangstemperaturen verfügbar sind, können diese mittels DSC, DMS (Dynamisch-mechanischer Analyse), dielektrischer Relaxations-Spektroskopie oder der Dilatometrie bestimmt werden. Eine übliche Methode ist die DSC-Messung zur Bestimmung der Glasübergangstemperatur der Homopolymere. Dazu wird das Homopolymer getrocknet, auf 120 °C erhitzt, schnell auf -100 °C abgekühlt und anschließend mit 20 °C/Minute auf 150 °C oder höher auf bis zu 300 °C erhitzt und die Daten der Glasübergangstemperatur ermittelt. Gemessen wird die Glasübergangstemperatur als Mittelwert.

Bevorzugt sind disubstituierte 4,4'-Di(oxabenzol)dialkylmethane der Formel I mit $R^1$ und $R^2$ jeweils Methyl, und $R^5$ und $R^6$ gleich und ausgewählt aus H, Methyl und Ethyl, insbesondere mit $R^5$ und $R^6$ gleich und ausgewählt aus H

und Methyl, und mit $R^3$ und $R^4$ jeweils unabhängig bivalent Ethylen oder Propylen mit n = 1 bis 6, bevorzugt n = 2 bis 4, und mit m = 1 bis 6, bevorzugt m = 2 bis 4, bevorzugt mit n = 2 und m = 2 oder mit n = 4 und m = 4 sowie Mischungen dieser.

Ein bevorzugtes mindestens di-funktionelles Monomer, das kein Urethan(meth)acrylat ist, ist ausgewählt aus (b) difunktionellen Acrylaten mit bivalenter alicyclischer Gruppe und difunktionellen Methacrylaten mit bivalenter alicyclischer Gruppe. Besonders bevorzugt sind (b) diese ausgewählt aus Tricyclodecandimethanoldiacrylat (TCDDA), Tricyclodecandimethanoldimethacrylat, Tricyclodecandiethanoldiacrylat, Tricyclodecandiethanoldimethacrylat und/oder Mischungen dieser (Teils synonym zu Bis(methacryloyloxymethyl)tetrahydrodicyclopentadien oder Bis(acryloyloxymethyl)tetrahydrodicyclopentadien).

Vorzugsweise weisen alle erfindungsgemäßen Monomere (a), (b), (c), (d), (e) und (f.1), (f.2) und/oder (f.3), ein mittleres Molekulargewicht (Gewichtsmittel) von kleiner 2000 g/mol auf, besonders bevorzugt weisen die Monomere (a), (b), (d), (e) und (f.1), (f.2) und/oder (f.3), ein mittleres Molekulargewicht von kleiner 1000 g/mol auf.

Auch bei der Auswahl der Monomere ist darauf zu achten, dass diese sich mit dem optional eingesetzten Füllstoff gut verbinden. In der Regel gehen Polyurethane, Acrylate, Polyester und andere Monomere keinen guten Verbund mit den eingesetzten Füllstoffen ein. Daher werden in der Regel die Füllstoffe für eine verbesserte Anbindung mit den Monomeren an den Oberflächen silanisiert oder hydrophobiert.

Sofern aufgrund der spezifischen dentalen Anwendung in der polymerisierbaren Zusammensetzung keine anorganischen Füllstoffe, bspw. aufgrund der angestrebten Viskosität der Zusammensetzung, eingesetzt werden können, besteht die Möglichkeit zur Reflexion der Strahlung, insbesondere diffusen Reflexion bzw. Streuung der eingestrahlten Strahlung, Farbstoffe oder Pigmente in der Zusammensetzung einzusetzen. Als Farbstoffe gelten Verbindungen, die in der polymerisierbaren Zusammensetzung löslich sind und vorzugsweise eine klare Lösung bilden.

Die erfindungsgemäßen, strahlenhärtbaren Zusammensetzungen können vorzugsweise mit einer Strahlenquelle die Licht im Vis-Bereich emittiert bestrahlt werden, besonders bevorzugt sind Strahlenquellen die von 360 bis 750 nm Strahlung emittieren, insbesondere bei ca. 385 nm, besonders bevorzugt bei ca. 405 nm. Besonders bevorzugt kann die erfindungsgemäße Zusammensetzung mit einer polychromatischen Strahlenquelle, wie einem DLP-Projektor, oder vorzugsweise mit einer monochromatischen Strahlenquelle, wie einem Laser-Projektor, im Vis-Bereich von 380 bis 660 nm bestrahlt werden.

Bei Zusatz dieser Pigmente und/oder Farbstoffe kann in der Zusammensetzung der Gehalt an Photoinitiator vermindert werden. Ein zu hoher Gehalt an Photoinitiator kann zu einem sogenannten "Overcuring" der bestrahlten Zusammensetzung, Ungenauigkeiten und/oder Geometrieveränderung führen, so dass die entsprechend hergestellten dentalen Teile nicht verwendbar sind.

Der Einsatz der optional verwendbaren erfindungsgemäßen anorganischen Füllstoffe, Pigmente oder Farbstoffe führt zu einer gleichmäßigen Streuung der Strahlenquellen, insbesondere der UV- und Vis-Strahlenquelle in der Monomermatrix der Zusammensetzung, so dass eine gleichmäßigere Härtung der Zusammensetzung angenommen wird. Im Ergebnis weisen die polymerisierten Zusammensetzungen erhöhte Werte bei der erzielten Brucharbeit auf.

Die erfindungsgemäße Zusammensetzung weist nach einem Belichten mit einer Strahlenquelle im Vis-Bereich, insbesondere von 385 bis 405 nm, bevorzugt nach dem Belichten in einem Stereolithographie-Verfahren und Erhalten einer polymerisierten Zusammensetzung, vorzugsweise in Form eines Rohlings, 3D-Formteils, dentalen prothetischen Teils, anatomischen Modells, anatomischen Tischmodells, dentalen Arbeitsmodells, dentalen Vollmodells, dentalen Stumpfmodells, anatomischen oder dentalen Sägeschnittmodells, insbesondere Situationsmodells, Gegenbissmodells, Funktionsmodells, Vormodells, Reparaturmodells, Präszisionsmodells, Meistermodells sowie präzisen Gegenbissmodells, anatomischen Modells zur Ersetzung des dentalen Gipsmodells des Gebisses, prothetischer Teile, orthopädischer Apparate oder dentaler Pre-Form, sowie optionaler Nachvergütung der polymerisierten Zusammensetzung mit einer Strahlenquelle, die folgenden Eigenschaften auf a) eine Biegefestigkeit von größer gleich 40 MPa, insbesondere größer gleich 75 MPa und/oder b) ein E-Modul von größer gleich 800 MPa, insbesondere größer 1500 MPa, insbesondere größer gleich 2000 MPa in Anlehnung an DIN EN ISO 20795-2, insbesondere in der Regel bei Raumtemperatur, vorzugsweise 23 °C plus/minus 2 °C, bevorzugt von Raumtemperatur bis (in Wasser) 55 °C. Vorzugsweise weist die strahlengehärtete Zusammensetzung, insbesondere als Formkörper oder Rohling, die nachfolgend beschriebenen Biegefestigkeiten und E-Module auf. Zur Definition der oben stehenden dentalen Modelle siehe Lehrbuch der Zahntechnik, Band 3, Quintessenz Verlag, A- Hohmann, W. Hielscher, 5. Aufl., 2012. Das Nachhärten bzw. Nachvergüten kann vorzugsweise z.B. mit einem Laborlichtgerät (HiLite Power 3D) oder im Lichtofen vorzugsweise mit einem Lichtspektrum von 390 - 540 nm erfolgen.

Optional kann die Zusammensetzung zusätzlich als (f.2) mindestens ein di-funktionelles Monomer, das kein Urethanacrylat oder Urethanmethacrylat ist, mindestens ein Polyetherdiacrylat, wie Poly(ethylenglycol)-Diacrylat, Poly(ethylenglycol)-Di(alkyl)acrylat, Poly(propylenglycol)-Diacrylat, Poly(propylenglycol)-Di(alkyl)acrylat oder eine Mischung von mindestens zwei der genannten Monomere enthalten. Bevorzugte Polyetherdiacrylate können ausgewählt sein aus Triethylenglycoldimethacrylat, Diethylenglycoldimethacrylat und/oder Tetraethylenglycoldimethacrylat. Alternativ oder zusätzlich kann die Zusammensetzung Diacrylate ausgewählt aus Decandioldi(meth)acrylat, Dodecandioldi(meth)acrylat, Hexyldecan-dioldi(meth)acrylat, Butandioldi(meth)acrylat oder Mischungen enthaltend mindestens eines der Acrylate umfassen.

Die Bezeichnung in Klammern in den Begriffen (Methyl)acrylat oder (Alkyl)acrylat bedeutet, dass die Acrylate als Acrylat oder Methylacrylat sowie alternativ als Alkylacrylat vorliegen können.

Als mono-funktionelles Monomer kann Hydroxyethylacrylat eingesetzt werden. Gleichfalls können Hydroxypropylacrylat, Hydroxyethylmethacrylat, Hydroxypropylmethacrylat und/oder Hydroxyethylacrylat optional als Mischung von mindestens zwei der vorgenannten Monomere eingesetzt werden.

Ferner kann die Zusammensetzung (d) mindestens ein mindestens difunktionelles Urethan(meth)acrylat enthalten, das ausgewählt ist aus difunktionellen Urethan(meth)acrylaten mit bivalenter Alkylen-Gruppe (difunktionellen Urethanacrylaten und/oder difunktionellen Urethanmethacrylaten). Der Gehalt kann von 0 bis 40 Gew.-% in der Gesamtzusammensetzung betragen, wobei der Gehalt vorzugsweise von 1 bis 35 Gew.-%, bevorzugt von 5 bis 35 Gew.-% beträgt und vorzugsweise Bestandteil des Anteils der ersten Monomere von 70 bis 99,99 % ist.

Das difunktionelle Urethan(meth)acrylat mit bivalenter Alkylen-Gruppe ist vorzugsweise ausgewählt aus linearen oder verzweigten mit einer bivalenten Alkylen-Gruppe funktionalisierten Urethandimethacrylaten, funktionalisierten Polyethern mit Alkylen-Gruppe(n), wie Bis(methacryloxy-2-ethoxycarbonylamino)-alkylen, Bis(methacryloxy-2-ethoxycarbonyl-amino)-substituierten Polyalkylenethern, vorzugsweise 1,6-Bis(methacryloxy-2-ethoxycarbonylamino)-2,4,4-trimethylhexan, UDMA bzw. HEMA-TDMI. Bevorzugt ist ein Bis(methacryloxy-2-ethoxycarbonylamino)-alkylen, wobei Alkylen lineare oder verzweigte C3 bis C20 umfasst, vorzugsweise C3 bis C6, wie besonders bevorzugt ein mit Methyl-Gruppen substiutiertes Alkylen, wie HEMA-TMDI. Das bivalente Alkylen umfasst vorzugsweise 2,2,4-Trimethylhexamethylen und/oder 2,4,4-Trimethylhexamethylen.

Optional können in der Zusammensetzung ein oder mehrere Füllstoffe enthalten sein, wie ein dotierter Siliziumdioxid Füllstoff, insbesondere ein Mischoxid von Zirkondioxid und Siliziumdioxid. Besonders bevorzugt sind u.a. agglomerierte Mischoxide, umfassend in Bezug auf die Gesamtzusammensetzung des Mischoxids 75 bis 99 Gew.-% Siliziumdioxid und von 1 bis 25 Gew.-% Zirkondioxid, insbesondere umfasst das Mischoxid von 85 bis 90 Gew.-% Siliziumdioxid und 10 bis 15 Gew.-% Zirkoniumdioxid, wobei es weiter bevorzugt ist, wenn die Primärteilchen der agglomerierten Oxidpartikel mikrokristalline Domänen von 4 bis 7 nm umfassen und vorteilhaft der Kristallinitätsindex 0.6 bis 0.7 - bestimmt nach der Methode von Windisch et al. (WO 01/30306A) - beträgt und die agglomerierten Oxidpartikel mit mindestens einem gegenüber mindestens einer Monomer- und/oder Polymerkomponente reaktiven organofunktionellen Silan oberflächenmodifiziert vorliegt. Diese erfindungsgemäß behandelten agglomerierten Oxidpartikel weisen hervorragende Eigenschaften in Abrasionsmessungen, bezüglich der Glanzzahl, eine hervorragende Transparenz und sehr gute Werte bei Messungen der Reflexion und Rauheit nach einem Zahnbürstentest auf.

Die Partikelgrößen der anorganischen Füllstoffe, wie des mindestens einen anorganischen Oxids, Mischoxids oder Dentalglases, bspw. umfassend Bariumaluminiumoxid, weisen für die vorliegenden Anwendungen im Durchschnitt einen mittleren Partikeldurchmesser von $d_{50}$ kleiner 10 μm auf, besonders bevorzugt weisen die Füllstoffe einen Partikeldurchmesser von circa 3 bis 70 nm auf, insbesondere von 10 bis 50 nm (Nanometer), optional können die Partikel aggregiert oder agglomeriert als Partikel mit bis zu 10 μm vorliegen. Die Primärpartikelgrößen der anorganischen Füllstoffe, die optional als agglomerierte und/oder aggregierte Primärpartikel vorliegen können, weisen im Durchschnitt einen Partikeldurchmesser von circa 3 bis 70 nm auf, insbesondere von 10 bis 50 nm. Bevorzugt weisen die Mischoxide von Zirkondioxid mit Siliziumdioxid eine Primärpartikelgröße von 3 bis 70 nm auf. Der Vorteil der sehr kleinen Partikeldurchmesser, die ggf. aggregiert und/oder agglomeriert vorliegen können, ist, dass das Licht an diesen Partikeln bei der Strahlenhärtung im Wesentlichen diffus gestreut wird und so zu einer verbesserten Härtung im Stereolitographie-Verfahren oder DLP-Verfahren führt.

Ferner ist es bevorzugt, wenn die Zusammensetzung nicht thixotrop ist. Zudem ist es besonders bevorzugt, wenn die Zusammensetzung eine Viskosität von kleiner 3000 m·Pas, insbesondere von 500 bis kleiner 2500 m·Pas, bevorzugt von 500 bis 2000 m·Pas, besonders bevorzugt von 500 bis 1600 m·Pas, aufweist. Die Viskosität wird vorzugsweise gemessen nach DIN 1342-2;2003-11 newtonsche Flüssigkeiten oder DIN 1342-3;2003-11 nicht newtonsche Flüssigkeiten mit einem Rheometer (Anton Par, Physiker NCR 301, Viskositätsbereiche 200-3000 m·Pas bei 100/s 23 °C). Die erfindungsgemäßen Zusammensetzungen weisen keine oder vorzugsweise nur eine geringe Thixotropie auf. Die hergestellten Zusammensetzungen sind strukturviskos, wobei es bevorzugt ist, wenn die Zusammensetzungen sowohl mit als auch ohne Füllstoffe strukturviskos sind. Nach einer weiteren Ausführungsform ist es bevorzugt, dass nahezu keine Viskositätsänderungen über einen längeren Lagerzeitraum auftreten. Ferner weisen die Zusammensetzungen eine sehr gute Reaktivität bei Belichtung mit einem Laser oder DLP-Projektor auf.

Besonders bevorzugte Photoinitiatoren umfassen alpha-Hydroxyphenylketon, Benzildimethylketal oder 2,4,6-Trimethylbenzoyldiphenylphosphinoxid, Phenyl-bis(2,4,6-trimethylbenzoyl)-Phosphinoxid, 2,4,6-Trimethylbenzoylphenylphosphinsäureethylester, und Gemische von mindestens zwei der Photoinitatoren, Phenylphosphinoxid-Kombination, Bisacylphosphinoxide (BAPO).

Typische Stabilisatoren umfassen 2,6-Di-tert.-butyl-4-methylphenol (BHT) oder Hydrochinonmonomethylether (MEHQ), 2-Hydroxy-4-methoxybenzophenon, HALS (Hindered Amine Light Stabilizers), Benzotriazol ultraviolet absorbers (UVAs) und Hydroxy Phenyl Triazines (HPT).

Nach einer weiteren bevorzugten Ausführungsform kann die Zusammensetzung umfassen:

(ii) 0,01 bis 2 Gew.-% Photoinitiator für den UV- und/oder Vis-Bereich oder ein Photoinitiatorsystem für den UV und/oder Vis-Bereich, und 0,01 bis 2 Gew.-% Stabilisator, und optional
(g) 0 bis 10 Gew.-%, insbesondere 0,01 bis 7,5 Gew.-% anorganische Füllstoffe umfassend anorganische Oxide oder anorganische Mischoxide und/oder Dentalgläser, insbesondere Zirkondioxid, Mischoxide von Zirkonoxid und Siliziumdioxid, Siliziumdioxid, wobei die Gesamtzusammensetzung 100 Gew.-% beträgt.

Gleichfalls Gegenstand der Erfindung sind Zusammensetzungen, optional umfassend Füllstoffe mit Primärpartikelgrößen der anorganischen Füllstoffe, die optional als agglomerierte und/oder aggregierte Primärpartikel vorliegen, von im Durchschnitt mit einem Partikeldurchmesser von circa 3 bis 70 nm, insbesondere von 10 bis 50 nm. Alternativ oder zusätzlich können übliche Füllstoffe mit Partikelgrößen von 0,4 bis 10 $\mu$m in der Zusammensetzung eingesetzt werden.

Ferner ist Gegenstand der Erfindung eine polymerisierte Zusammensetzung sowie ein entsprechender 3D-Formkörper als auch die nachfolgend genannten dentalen Modelle, Schienen, KFO-Apparate und prothetischen Formteile oder Rohlinge, wobei die polymerisierte Zusammensetzung alternativ oder kumulativ i) a) eine Biegefestigkeit von größer gleich 75 MPa (in Anlehnung an DIN EN ISO 20795-2) aufweist, insbesondere größer gleich 80 MPa, bevorzugt größer gleich 90 MPa gemessen (in Anlehnung an DIN EN ISO 139), und/oder b) ein E-Modul von größer gleich 2000 MPa (in Anlehnung an DIN EN ISO 20795-2) , und/oder

ii) a) eine Biegefestigkeit von größer gleich 70 MPa (in Anlehnung an DIN EN ISO 20795-2), insbesondere gemessen in Wasser bei 37 °C, aufweist und/oder b) ein E-Modul von größer gleich 2000 MPa (in Anlehnung an DIN EN ISO 20795-2), insbesondere gemessen in Wasser bei 37 °C, und/oder
iii) a) eine Biegefestigkeit von größer gleich 50 MPa (in Anlehnung an DIN EN ISO 20795-2), insbesondere gemessen in Wasser bei 45 °C, aufweist und/oder b) ein E-Modul von größer gleich 1500 MPa (in Anlehnung an DIN EN ISO 20795-2), insbesondere gemessen in Wasser bei 45 °C, und/oder
iv) a) eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C, die insbesondere erhältlich sind durch Bestrahlung einer polymerisierbaren Zusammensetzung.

Dabei ist es besonders bevorzugt, wenn die polymerisierten Zusammensetzungen einen Schrumpf kleiner 7 %, vorzugsweise einen Schrumpf kleiner gleich 6,8 %, bevorzugt kleiner gleich 6,5 %, besonders bevorzugt kleiner gleich 6,0 % aufweisen (bestimmt nach Watts, Dent. Mater 7: 281-286, Okt. 1991, auch Bonded Disc-Methode genannt, bei Raumtemperatur, Translux Energy, 60 s Belichtung).

Ferner ist Gegenstand der Erfindung ein Rohling in Form eines dreidimensionalen Formkörpers einer polymerisierten Zusammensetzung geeignet zur Herstellung dentaler prothetischer Teile, orthopädischer Apparate oder dentaler Pre-Forms, wobei der Rohling a.1) eine Biegefestigkeit von größer gleich 75 MPa (in Anlehnung an DIN EN ISO 20795-2) aufweist und/oder b.1) ein E-Modul von größer gleich 2000 MPa (in Anlehnung an DIN EN ISO 20795-2) aufweist und optional

a.2) eine Biegefestigkeit von größer gleich 50 MPa (in Anlehnung an DIN EN ISO 20795-2), insbesondere gemessen in Wasser bei 45 °C, aufweist und/oder b.2) ein E-Modul von größer gleich 1500 MPa (in Anlehnung an DIN EN ISO 20795-2), insbesondere gemessen in Wasser bei 45 °C, aufweist und optional
iv) a) eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C.

Ebenso Gegenstand der Erfindung ist die Verwendung einer Zusammensetzung zur Herstellung anatomischer Modelle, anatomischer Tischmodelle, anatomischer Modelle zur Ersetzung des dentalen Gipsmodells des Gebisses, prothetischer Teile, dentaler prothetischer Teile, orthopädischer Apparate, Alignern, Zahnschienen oder dentaler Pre-Forms, sowie die Verwendung einer erfindungsgemäßen Zusammensetzung in einem Rapid-Prototyping- oder in einem Rapid-Manufacturing- oder Rapid-Tooling-Verfahren. Bevorzugt ist eine eine Strahlenhärtung der Zusammensetzung mittels Laserstrahlen, LED-Leuchtmitteln oder DLP-Projektoren.

Ferner ist Gegenstand der Erfindung die Verwendung zur Herstellung dentaler prothetischer Teile umfassend Prothesenbasis oder Teile davon, künstliche Zähne, Zahnbogen mit mindestens zwei bis 16 interdental werkstoffeinstückig verbundenen künstlichen Zähnen, Kronen, provisorische Kronen, Totalprothesen, Vollkronen, Schienen für kieferorthopädische Korrekturen (ähnlich *Invisalign*), dentale Brücken, Abutments, Suprastrukturen, dentale Stege, Inlays,

Onlays, orthopädische Apparate, wie Aufbissschienen, dentale Pre-Forms von künstlichen Zähne, Bohrschablonen für die Implantologie, Mouthguards, und/oder Implantate. Unter Dentalprodukten werden vorliegend insbesondere Dentalprodukte verstanden, die aus polymerisierbaren Zusammensetzungen herstellbar sind, wie z.B. nicht abschließend Totalprothesen, provisorische Kronen und Brücken, Inlays, Onlays, Vollkronen, Aufbissschienen, Bohrschablonen für die Implantologie, Schienen für kieferorthopädische Korrekturen (ähnlich *Invisalign*), Mouthguards, künstliche Zähne.

Zur Erfüllung hoher ästhetischer Ansprüche müssen im Dentalbereich verwendbare Zusammensetzungen zur Herstellung von definitivem Zahnersatz, wie z.B. Arbeitsmodellen, KFO Modellen, Bohrschablonen, Provisorien sowie Schienen eine hohe Transparenz aufweisen. Diese Transparenz wird in der Regel durch eine optimale Anpassung der Brechzahlen der Füllstoffe und der Polymermatrix erreicht. Hierbei sind jedoch aufgrund verschiedener physikalischer und chemischer Randbedingungen sowohl bei der Auswahl der Füllstoffe als auch der Monomere sehr enge Grenzen gesetzt.

Nach einer weiteren alternativen Ausführungsform sind polymerisierte Zusammensetzungen oder Rohlinge erhältlich, insbesondere strahlengehärtete Zusammensetzungen, insbesondere UV/Vis-gehärtete Zusammensetzungen, die vorzugsweise zusätzlich allseitig strahlengehärtet wurden, die die vorstehend genannten Eigenschaften in Bezug auf ihre Biegefestigkeit und/oder E-Modul angelehnt an DIN EN ISO 20795-2 aufweisen. Unter einer zusätzlich allseitigen Strahlenhärtung wird beispielsweise eine Nachvergütung im 3D-Lichtofen verstanden.

Dabei umfassen die folgenden Verfahren - Rapid-Prototyping oder Rapid-Manufacturing, ein Verfahren zur Herstellung von Werkstücken, wie ein dentales prothetisches Teil, oder Rapid-Tooling, ein Verfahren zur Herstellung von Werkzeugen - jeweils Stereolithographie-Verfahren und DLP-Verfahren. Optional kann nach dem Aushärten der polymerisierbaren Zusammensetzung in den vorgenannten Verfahren eine Nachvergütung mit UV-, Vis- oder UV/Vis-Licht erfolgen. Vorzugsweise erfolgt eine Nachvergütung der polymerisierten Zusammensetzung oder der dentalen prothetischen Teile, der orthopädischen Apparate oder dentalen Pre-Forms oder Rohlingen gleichzeitig von mindestens drei Seiten, bevorzug von fünf bis sechs Seiten, wie dies in einem Lichtofen möglich ist. Alternativ kann die polymerisierte Zusammensetzung zusätzlich oder alternativ getempert werden.

Der Zusammensetzung können zusätzlich Farbpigmente zur Einstellung der Farbe zugesetzt werden. Zudem können der Zusammensetzung rote Fasern zugesetzt werden, um Adern der Gingiva zu imitieren. Beispielshaft geeignete Farbpigmente sind: PV Echtrot - CAS 4948-15-6, Indischblau 220943 - CAS 68186-87-8, Echtschwarz 100 - 68186-91-4, Kronos 2220 - CAS 13463-67-7 und Lichtgelb 3R - CAS 68186-90-3.

In den polymerisierten Zusammensetzungen können Schichtstärken im Bereich von 5 µm, insbesondere von 25 µm bis 250 µm pro Aushärteschicht erzielt werden. Die Druckschichten liegen besonders bevorzugt bei 30 µm, 50 µm, 70 µm, 100 µm, 120 µm und 170 µm.

Eine hohe Transparenz kann durch die optimale Auswahl der Rezepturkomponenten in Bezug auf ihre Brechungsindizes erreicht werden.

Als Photoinitiatoren kommen beispielsweise Benzoinalkylether oder -ester, Benzilmonoketale, Acylphosphinoxide oder aliphatische und aromatische 1,2-Diketoverbindungen, wie beispielsweise 2,2-Diethoxyacetophenon, 9,10-Phenanthrenchinon, Diacetyl, Furil, Anisil, 4,4'-Dichlorbenzil und 4,4'-Dialkoxybenzil oder Campherchinon, in Frage. Die Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein oder in Kombination mit einem Initiator für sichtbares Licht eingesetzt werden.

Besonders bevorzugte Photoinitiatoren und/oder Initiatorsysteme umfassen a) mindestens einen radikalischen Photoinitiator, insbesondere mindestens ein Peroxid und/oder eine Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid, oder ein alpha-Hydroxyketon, Campherchinon, Acylphosphinoxid. Optional können zudem Stabilisatoren zugesetzt werden und optional b) mindestens ein Co-Initiator wie ein Amin, in der Regel ein tert-Amin, insbesondere mindestens ein aromatisches Amin, wie N,N-Dimethyl-p-toluidin, N,N-Dihydroxyethyl-p-toluidin und/oder p-Dibenzylaminobenzoesäurediethylester. Besonders bevorzugte Photoinitiatoren umfassen alpha-Hydroxyphenylketon, Benzildimethylketal oder 2,4,6-Trimethylbenzoyldiphenylphosphinoxid, Phenyl-bis(2,4,6-trimethylbenzoyl)-Phosphinoxid, 2,4,6-Trimethylbenzoylphenylphosphinsäureethylester, und Gemische von mindestens zwei der Photoinitatoren, Phenylphosphinoxid-Kombination, Bisacylphosphinoxide (BAPO).

Typische Stabilisatoren umfassen 2,6-Di-tert.-butyl-4-methylphenol (BHT) oder Hydrochinonmonomethylether (MEHQ), 2-Hydroxy-4-methoxybenzophenon), HALS (Hindered Amine Light Stabilizers), Benzotriazol ultraviolet absorbers (UVAs) und Hydroxy Phenyl Triazines (HPT).

Die Erfindung wird durch die nachfolgende Beispiele näher erläutert, ohne die Erfindung auf diese Ausführungsbeispiele zu beschränken.

Mit der erfindungsgemäßen Zusammensetzung können Werkstücke, Rohlinge bzw. dreidimensionale Formkörper mit sehr guter geometrischer Präzision/Auflösung gedruckt werden. Die erfindungsgemäßen Formkörper weisen sehr gute mechanische Eigenschaften selbst bei erhöhten Temperaturen auf. Ferner ist eine gute Farbstabilität bei den Werkstücken zu beobachten.

**Ausführungsbeispiele:**

Allgemeines Herstellungsbeispiel: die Initiatoren werden in (Octahydro-4,7-metano-1H-indenyl)methyl acrylat oder Tricyclodecan Dimethanol-Diacrylat vorgelöst. Anschließend werden die anderen Monomere zugegeben und die Mischung wird homogenisiert. Es können Pigmentkonzentrate oder Pigmente zugegeben werden. Die Zusammensetzung wird dann vorzugsweise homogenisiert. Die hergestellte Zusammensetzung ist mit einem 3D-Drucker verarbeitbar. Hierbei ist zu beachten, dass die lichtsensiblen Initiatoren in Verbindung mit dem Umgebungslicht zu einer ungewollten Polymerisation reagieren können (die Zusammensetzung wird vorzugsweise unter geeigneten Maßnahmen in das Druckbad überführt). Belichtet wird bei 385 - 405 nm und nachgehärtet bzw. nachvergütet wird z.B. mit einem Laborlichtgerät HiLite Power 3D.

Mit der hergestellten Mischung werden auf einem 3D-Präzisionsdrucker mit der Wellenlänge 405 nm (Cara Print 4.0) Prüfkörper gemäß ISO 20795-2 (50 $\mu$m) für die nachfolgenden Abprüfungen verdruckt. Nach dem Druckprozess werden die Prüfkörper mit Isopropanol abgespült und einem Nachvergütungsprozess unterzogen. Dieser erfolgt durch beidseitiges Belichten jeweils 3 bis 5 min. oder gemäß Herstellerangaben in einer Laborlichtlampe HiLite Power 3D, 200 W (Kulzer GmbH). Eigenschaften der erfindungsgemäßen Mischung für Modell Materialien, abgeprüft nach DIN EN ISO 20795-2 bzw. angelehnt an diese Norm:

**Tabelle 1: Vergleichsbeispiel TG**

| | Funktional | TG | **VG1** | **VG2** |
|---|---|---|---|---|
| Tris(2-Hydroxyethyl)isocyanurat Triacrylat | 3 | 270 | | 17,7 |
| ethoxyliertes (2) Bisphenol A Dimethacrylat | 2 | 115 | 30 | 30 |
| (Octahydro-4,7-metano-1H-indenyl)methylacrylat | 1 | 35 | 17,7 | |
| Tricyclodecan-dimethanol-diacrylat | 2 | 185 | 10 | 10 |
| aliphatisches Urethan-acrylat | 2 | 148 | | |
| Amin modifiziertes Polyetheracrylat | 4 | 25 | 35 | 35 |
| ethoxyliertes (4) Bisphenol A Diacrylat | 2 | 60 | 6 | 6 |
| Stabilisator/Photoinitiator | | | 0,5 bis 2,0 | 0,5 bis 2,00 |
| | | | | |
| Biegefestigkeit in MPa | | | 35,4 | 54,2 |
| E-Modul in MPa | | | 1081 | 1761 |
| Biegebruch in MPa $m^{1/2} \geq 1,1$ | | | 0,67 | 0,47 |
| Brucharbeit in $J/m^2$ >250 | | | 75,6 | 33,49 |

Die Vergleichsbeispiele 1 und 2 zeigen, dass allein ein hoher Anteil an vierfach-Vernetzern kein Garant für gute mechanische Eigenschaften ist, da die Vergleichsbeispiele bereits bei Raumtemperatur keine akzeptablen mechanischen Eigenschaften aufweisen. Zwar weisen die Zusammensetzungen der Vergleichsbeispiele eine für 3D-Druckanwendungen gut verwendbare Viskosität auf, doch die Anforderungen an die mechanischen Eigenschaften in Anlehnung an ISO-20795 werden nicht erreicht.

**Tabelle 2a: VG3 und Beispiele 1 und 2**

| | Zusammensetzungen | | |
|---|---|---|---|
| | VG3 | 1 | 2 |
| Tris (2-hydroxyethl)isocyanurat-Triacrylat | | | |
| ethoxyliertes (2) Bisphenol A Dimethacrylat | 30 | 36 | 42,25 |
| (Octahydro-4,7-metano-1H-indenyl)methyl acrylat | 19,86 | 19,86 | 19,86 |
| Tricyclodecan-dimethanoldiacrylat | 10 | 10 | 10 |
| Carboxy-Acrylat* | 28,25 | 6,25 | |
| ethoxyliertes (4)Bisphenol A Diacrylat | 11,25 | | |
| UDMA | | 27,25 | 27,25 |
| Rechnerischer TG | 79 | 107 | 111 |
| * TG: 39 °C | | | |

**Tabelle 2b: Biegefestigkeit und E-Modul**

| Lagerung | Prüfung | | Ausprüfung Zusammensetzungen | | |
|---|---|---|---|---|---|
| | | | VG3 | 1 | 2 |
| Lagerung trocken ISO/20795 | trocken | Biegefestigkeit in [MPa] | 113,3 | 82 | 81,3 |
| | | E-Modul in [MPa] | 2880 | 2494 | 2319 |
| Lagerung trocken ISO/20795 | in Wasser (37 °C) | Biegefestigkeit in [MPa] | 68,7 | 76,6 | 63,2 |
| | | E-Modul in [MPa] | 2131 | 2012 | 1797 |
| Lagerung trocken ISO/20795 | in Wasser (45 °C) | Biegefestigkeit in [MPa] | 45,6 | 59,2 | 55,7 |
| | | E-Modul in [MPa] | 1329 | 1624 | 1528 |
| Lagerung trocken ISO/20795 | in Wasser (55 °C) | Biegefestigkeit in [MPa] | 21,1 | 46,8 | 43,9 |
| | | E-Modul in [MPa] | kein | 1121 | 987 |

**Tabelle 3a: Beispiele 3 bis 6**

| | Zusammensetzungen | | | |
|---|---|---|---|---|
| | 3 | 4 | 5 | 6 |
| Tris (2-hydroxyethl)isocyanurat-Triacrylat | 13,34 | 11 | 10 | 12 |
| ethoxyliertes (2) Bisphenol A Dimethacrylat | 40 | 40 | 45 | 40 |
| (Octahydro-4,7-metano-1H-indenyl)methyl acryl at | 13,34 | 11 | 10 | 12 |
| Tricyclodecan-dimethanol-diacrylat | 13,34 | 11 | 10 | 12 |
| Carboxy-Acrylat | | | | |
| Ethoxyliertes (4) Bisphenol A Diacrylat | | | | |
| UDMA | 17,8 | 24,7 | 22,7 | 22 |
| Rechnerischer TG | 137 | 135,5 | 133 | 130 |

**Tabelle 3b: Beispiele 3 bis 6**

| Lagerung | Prüfung | Mechanik in Anlehnung an ISO-20795 | **3** | **4** | **5** | **6** |
|---|---|---|---|---|---|---|
| Lagerung trocken ISO/ 20795 | trocken | Biegefestigkeit in [MPa] | 85,5 | 93 | 105,5 | 104,3 |
| | | E-Modul in [MPa] | 2592 | 2841 | 2883 | 2726 |
| Lagerung trocken ISO/ 20795 | in Wasser (37 °C) | Biegefestigkeit in [MPa] | 72,2 | 87,5 | 88,1 | 84,5 |
| | | E-Modul in [MPa] | 2134 | 2447 | 2344 | 2264 |
| Lagerung trocken ISO/ 20795 | in Wasser (45 °C) | Biegefestigkeit in [MPa] | 62,1 | 65,1 | 70,6 | 68,5 |
| | | E-Modul in [MPa] | 1799 | 1831 | 1958 | 1813 |
| Lagerung trocken ISO/ 20795 | in Wasser (55°C) | Biegefestigkeit in [MPa] | 45,8 | 58,2 | 54,8 | 51,1 |
| | | E-Modul in [MPa] | 1120 | 1428 | 1346 | 1194 |

**Tabelle 4a: Thermostabilität erfindungsgemäßer Zusammensetzungen**

| | **Beispiel 7** | **Beispiel 8** |
|---|---|---|
| Rechnerischer TG | 135 | 201 |

**Thermostabilität**

| Lagerung | Prüfung | Mechanik in Anlehnung an ISO-20795 | | |
|---|---|---|---|---|
| Lagerung trocken | trocken | Biegefestigkeit in [MPa] | 84,2 | 95,3 |
| | | E-Modul in [MPa] | 2615 | 2859 |
| Lagerung trocken | in Wasser (37 °C) | Biegefestigkeit in [MPa] | 81,2 | 79,4 |
| | | E-Modul in [MPa] | 2185 | 2412 |
| Lagerung trocken | in Wasser (45 °C) | Biegefestigkeit in [MPa] | 58,2 | 75,7 |
| | | E-Modul in [MPa] | 1559 | 1993 |
| Lagerung trocken | in Wasser (55°C) | Biegefestigkeit in [MPa] | 44,9 | 60,2 |
| | | E-Modul in [MPa] | 940 | 1582 |
| Schrumpf nach Watts | | [%] | 5,5 | 6,76 |
| Viskosität [m·Pas] | | | 1390 | 2224 |

**Tabelle 4b: Erfindungsgemäße Zusammensetzungen**

| **Monomere** | Rechnerischer TG | **Beispiel 7** | **Beispiel 8** |
|---|---|---|---|
| | **TG** | 135 °C | 201 °C |
| | | Anteil in Gew.-% | Anteil in Gew.-% |
| Aliphatisches Urethandimethacrylat | 148 | 27,7 | 32,5 |
| ethoxyliertes (2) Bisphenol A Dimethacrylat | 105 | 40 | |
| Tris(2-hydroxyethl)isocyanurat Triacrylate | 270 | 10 | 32,5 |
| (Octahydro-4,7-metano-1H-indenyl)methylacrylat | 35 | 10 | |
| Tricyclodecan-dimethanol-diacrylat | 185 | 10 | 32,5 |

## Patentansprüche

1. Polymerisierbare, strahlenhärtbare Zusammensetzung umfassend

(i) Monomere und
(ii) mindestens eine weitere Komponente,

**dadurch gekennzeichnet, dass**

(i) die Monomere mindestens (a) ein erstes Monomer mit einem TG (Glasübergangspunkt) des Homopolymers dieses ersten Monomers von größer gleich 120 °C umfassen und mindestens (b) ein zweites Monomer mit einem TG (Glasübergangspunkt) des Homopolymers des zweiten Monomers von kleiner gleich 100 °C umfassen, wobei mindestens eines des mindestens einen ersten Monomers oder des mindestens einen zweiten Monomers eine alicyclische Gruppe aufweist, und , wobei in der Zusammensetzung vorliegen
(i) 40 bis 99,99 Gew.-% (a) mindestens ein erstes Monomer oder eine Mischung von ersten Monomeren mit einem TG (Glasübergangspunkt) des jeweiligen Homopolymers des entsprechenden ersten Monomers von größer gleich 120 °C vorliegen und umfasst mindestens ein difunktionelles Acrylat mit bivalenter alicyclischer Gruppe und/oder difunktionelles Methacrylat mit bivalenter alicyclischer Gruppe
und mindestens ein difunktionelles Urethan(meth)acrylat ausgewählt aus difunktionellen Urethan(meth)acrylaten mit bivalenter Alkylen-Gruppe, und

- bis 60 Gew.-% mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C vorliegen, und

die zweiten Monomere umfassen mindestens ein monofunktionelles Acrylat mit alicyclischer Gruppe und/oder monofunktionelles Methacrylat mit alicyclischer Gruppe, und
(ii) 0,01 bis 5 Gew.-% mindestens einer weiteren Komponente, umfassend 0,01 bis 5 Gew.-% der mindestens einen weiteren Komponente vorliegen, die mindestens einen Photoinitiator für den UV- und/oder den Vis-Bereich oder ein Photoinitiatorsystem für den UV- und/oder den Vis-Bereich und optional mindestens einen Stabilisator für den UV- und/oder Vis-Bereich und optional mindestens ein Pigment und/oder Farbstoff sowie weitere übliche Additive,

wobei die Gesamtzusammensetzung 100 Gew.-% beträgt und, insbesondere wobei die Viskosität der Zusammensetzung bei Raumtemperatur (ca. 20 °C bis 23 °C) kleiner gleich 3000 m·Pas, insbesondere von 500 bis kleiner 2500 m·Pas ist.

2. Zusammensetzung nach Anspruch 1, **dadurch gekennzeichnet, dass** die Viskosität der Zusammensetzung bei Raumtemperatur (ca. 20 °C bis 23 °C) kleiner gleich 3000 m·Pas, insbesondere von 500 bis kleiner 2500 m·Pas beträgt.

3. Zusammensetzung nach Anspruch 1 oder 2, **dadurch gekennzeichnet, dass** (i) die Monomere umfassen

(a) mindestens ein aus 1,3,5-Tris(hydroxyalkyl)isocyanurat abgeleitetes Triacrylat, wobei der Hydroxyalkyl-Rest jeweils unabhängig 1 bis 8 C-Atome umfasst und optional
(b) difunktionelle Acrylate mit bivalenter alicyclischer Gruppe und/oder difunktionelle Methacrylate mit bivalenter alicyclischer Gruppe, und optional
(c) mindestens ein monofunktionelles Acrylat mit alicyclischer Gruppe und/oder monofunktionelles Methacrylat mit alicyclischer Gruppe.

4. Zusammensetzung nach Anspruch 3, **dadurch gekennzeichnet, dass** (i) die Monomere (a) mindestens ein erstes Monomer oder eine Mischung von ersten Monomeren mit einem TG (Glasübergangspunkt) des Homopolymers des jeweiligen ersten Monomers von größer gleich 120 °C umfassen, wobei das erste Monomer oder die Mischung von ersten Monomeren mindestens einen Diacrylatester basierend auf einem Tricyclodecan-dialkanol, mit Alkanol umfassend 1 bis 6 C-Atome, oder eine Mischung dieser umfasst, und

(b) mindestens ein zweites Monomer oder eine Mischung von zweiten Monomeren mit jeweils einem TG (Glasübergangspunkt) des Homopolymers des jeweiligen zweiten Monomers von kleiner gleich 100 °C umfassen,

wobei das zweite Monomer oder die Mischung von zweiten Monomeren mindestens einen mono-funktionellen Acrylatester basierend auf einem Tricyclodecanalkanol, mit Alkanol umfassend 1 bis 6 C-Atome, oder eine Mischung dieser und optional mindestens ein difunktionelles Urethan(meth)acrylat ausgewählt aus difunktionellen Urethan(meth)acrylaten mit bivalenter Alkylen-Gruppe, umfasst, wobei vorzugsweise

(i) in der Zusammensetzung 15 bis 25 Gew.-% mono-funktioneller Acrylatester basierend auf einem Tricyclodecanalkanol, mit Alkanol umfassend 1 bis 6 C-Atome, und optional 5 bis 15 Gew.-% Diacrylatester basierend auf einem Tricyclodecan-dialkanol, mit Alkanol umfassend 1 bis 6 C-Atome, vorliegen, wobei die Gesamtzusammensetzung 100 Gew.-% beträgt, oder
(ii) 5 bis 15 Gew.-% Diacrylatester basierend auf einem Tricyclodecan-dialkanol, mit Alkanol umfassend 1 bis 6 C-Atome, und 15 bis 25 Gew.-% mono-funktioneller Acrylatester basierend auf einem Tricyclodecanalkanol, mit Alkanol umfassend 1 bis 6 C-Atome, vorliegen, insbesondere in (ii) ohne die Gegenwart von aus 1,3,5-Tris(hydroxyalkyl)isocyanurat abgeleitetem Triacrylat, wobei die Gesamtzusammensetzung 100 Gew.-% beträgt, oder
(iii) 15 bis 45 Gew.-% Mischung umfassend mono-funktionellen Acrylatester basierend auf einem Tricyclodecanalkanol, mit Alkanol umfassend 1 bis 6 C-Atome, und Diacrylatester basierend auf einem Tricyclodecan-dialkanol, mit Alkanol umfassend 1 bis 6 C-Atome, insbesondere in (iii) ohne die Gegenwart von aus 1,3,5-Tris(hydroxyalkyl)isocyanurat abgeleitetem Triacrylat, wobei die Gesamtzusammensetzung 100 Gew.-% beträgt.

**5.** Zusammensetzung nach einem der Ansprüche 1 bis 4, **dadurch gekennzeichnet, dass**

(a) ausgewählt ist aus Tricyclodecandimethanoldiacrylat, Tricyclodecandimethanoldimethacrylat, Tricyclodecandiethanoldiacrylat, Tricyclodecandiethanoldimethacrylat und/oder Mischungen dieser.

**6.** Zusammensetzung nach Anspruch 4, **dadurch gekennzeichnet, dass** (i) das erste Monomer umfasst (f.1) mindestens ein mono-, tri-, tetra- oder multi-funktionelles Monomer, das insbesondere kein Urethan(meth)acrylat ist, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern.

**7.** Zusammensetzung nach Anspruch 4, **dadurch gekennzeichnet, dass** (i) das zweite Monomer umfasst

(c) mindestens ein monofunktionelles Acrylat mit alicyclischer Gruppe und/oder mono-funktionelles Methacrylat mit alicyclischer Gruppe, insbesondere ausgewählt aus (Octahydro-4,7-methano-1H-indenyl)methanolacrylat, (Octahydro-4,7-methano-1H-indenyl)methanolmethacrylat, (Octahydro-4,7-methano-1H-indenyl)ethanolacrylat und (Octahydro-4,7-methano-1H-indenyl)ethanolmethacrylat,
(f.2) mindestens ein mono-, tri-, tetra- oder multi-funktionelles Monomer, das insbesondere kein Urethan(meth)acrylat ist, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern.

**8.** Zusammensetzung nach einem der Ansprüche 1 bis 7, **dadurch gekennzeichnet, dass** in der Zusammensetzung vorliegen (i) Monomere umfassend

- 70 bis 99,99 Gew.-% (a) erstes Monomer oder Mischung von ersten Monomeren mit einem TG (Glasübergangspunkt) des jeweiligen Homopolymers des entsprechenden ersten Monomers von größer gleich 120 °C, wobei das erste Monomer oder die Mischung von ersten Monomeren umfasst mindestens ein difunktionelles Acrylat mit bivalenter alicyclischer Gruppe und/oder mindestens ein difunktionelles Methacrylat mit bivalenter alicyclischer Gruppe und optional mindestens ein difunktionelles Urethan(meth)acrylat ausgewählt aus difunktionellen Urethan-(meth)acrylaten mit bivalenter Alkylen-Gruppe, und
- bis 30 Gew.-% mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C, wobei das zweite Monomer oder die Mischung von zweiten Monomeren umfasst mindestens ein Monomer oder eine Mischung von zweiten Monomeren mit einem TG des jeweiligen Homopolymers von größer gleich 30 °C, bevorzugt umfasst sind zweite Monomere mit einem TG im Bereich von größer gleich 30 °C bis kleiner gleich 100 °C, besonders bevorzugt sind zweite Monomere mit einem TG im Bereich von größer gleich 50 °C bis kleiner gleich 100 °C, und

- 0,01 bis 5 Gew.-% der mindestens einen weiteren Komponente umfassend mindestens einen Photoinitiator für den UV- und/oder den Vis-Bereich oder ein Photo initiatorsystem für UV- und/oder Vis-Bereich und optional mindestens einen Stabilisator für den UV- und/oder Vis-Bereich und optional mindestens ein Pigment und/oder Farbstoff sowie weitere übliche Additive, wobei die Gesamtzusammensetzung 100 Gew.-% beträgt.

**9.** Zusammensetzung nach einem der Ansprüche 1 bis 89, **dadurch gekennzeichnet, dass** in der Zusammensetzung vorliegen (i) Monomere umfassend

- 75 bis 99,99 Gew.-% (a) erstes Monomer oder Mischung von ersten Monomeren mit einem TG (Glasübergangspunkt) des jeweiligen Homopolymers des entsprechenden ersten Monomers oder der Mischung von ersten Monomeren von größer gleich 120 °C, wobei das erste Monomer oder die Mischung von ersten Monomeren umfasst mindestens ein difunktionelles Acrylat mit bivalenter alicyclischer Gruppe und/oder mindestens ein difunktionelles Methacrylat mit bivalenter alicyclischer Gruppe und optional mindestens ein difunktionelles Urethan(meth)acrylat ausgewählt aus difunktionellen Urethan(meth)acrylaten mit bivalenter Alkylen-Gruppe, und
- bis 25 Gew.-% mindestens (b) ein zweites Monomer oder eine Mischung von zweiten Monomeren mit einem TG (Glasübergangspunkt) des jeweiligen Homopolymers des entsprechenden zweiten Monomers von kleiner gleich 100 °C, wobei das zweite Monomer oder die Mischung von zweiten Monomeren umfasst mindestens ein monofunktionelles Acrylat mit alicyclischer Gruppe und/oder monofunktionelles Methacrylat mit alicyclischer Gruppe, mit
- 0,01 bis 5 Gew.-% der mindestens einen weiteren Komponente umfassend mindestens einen Photoinitiator für den UV- und/oder Vis-Bereich oder ein Photoinitiatorsystem für den UV- und/oder Vis-Bereich und optional mindestens einen Stabilisator für den UV- und/oder Vis-Bereich und optional mindestens ein Pigment und/oder Farbstoff sowie weitere übliche Additive,

wobei die Gesamtzusammensetzung 100 Gew.-% beträgt, und, insbesondere wobei die Viskosität der Zusammensetzung bei Raumtemperatur (ca. 20 °C bis 23 °C) kleiner gleich 3000 m·Pas, insbesondere von 500 bis kleiner 2500 m·Pas ist.

**10.** Zusammensetzung nach einem der Ansprüche 1 bis 9, **dadurch gekennzeichnet, dass** die (i) Monomere und die (ii) mindestens eine weitere Komponente nach folgender Formel in der Zusammensetzung vorliegen,

$$1/TG(gesamt) = w1/TG_{(1)} + w2/TG_{(2)} + w3/TG_{(3)} + \text{optional } w4/TG_{(4)} + \text{optional } w5/TG_{(5)} + \text{optional } wn/TG_{(n)},$$

mit w1, w2, w3, w4, w5 und wn jeweils Gewichtsanteil des Monomers in der Gesamtmischung der ersten und zweiten Monomere und optional dritten Monomere von 100 Gew.-%, mit n = 6 bis 50, wobei TG größer gleich 100 °C beträgt, und die

- Viskosität der Zusammensetzung bei Raumtemperatur (ca. 20 °C bis 23 °C) kleiner gleich 3000 m·Pas, insbesondere von 500 bis kleiner 2500 m·Pas ist.

**11.** Polymerisierte Zusammensetzung nach einem der Ansprüche 1 bis 10, **dadurch gekennzeichnet, dass** die (i) Monomere nach folgender Formel in der Zusammensetzung vorliegen,

$$1/TG(gesamt) = w1/TG_{(1)} + w2/TG_{(2)} + w3/TG_{(3)} + \text{optional } w4/TG_{(4)} + \text{optional } 5/TG_{(5)} + \text{optional } wn/TG_{(n)},$$

mit w1, w2, w3, w4, w5 und wn jeweils Gewichtsanteil des Monomers in der Gesamtmischung der ersten und zweiten Monomere und optional des dritten Monomers von 100 Gew.-%, mit n = 6 bis 50, wobei TG größer gleich 100 °C beträgt, und die polymerisierte Zusammensetzung eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 800 MPa (in Anlehnung an DIN EN ISO 20795-2), insbesondere gemessen in Wasser bei 55 °C.

**12.** Polymerisierte Zusammensetzung nach einem der Ansprüche 1 bis 10 oder nach Anspruch 11, **dadurch gekennzeichnet, dass** die polymerisierte Zusammensetzung alternativ oder kumulativ

i) a) eine Biegefestigkeit von größer gleich 75 MPa (in Anlehnung an DIN EN ISO 20795-2) aufweist und/oder b) ein E-Modul von größer gleich 2000 MPa (in Anlehnung an DIN EN ISO 20795-2), und/oder
ii) a) eine Biegefestigkeit von größer gleich 70 MPa (in Anlehnung an DIN EN ISO 20795-2), insbesondere gemessen in Wasser bei 37 °C, aufweist und/oder b) ein E-Modul von größer gleich 2000 MPa (in Anlehnung an DIN EN ISO 20795-2), insbesondere gemessen in Wasser bei 37 °C aufweist, und/oder
iii) a) eine Biegefestigkeit von größer gleich 50 MPa (in Anlehnung an DIN EN ISO 20795-2), insbesondere gemessen in Wasser bei 45 °C, aufweist und/oder b) ein E-Modul von größer gleich 1500 MPa (in Anlehnung an DIN EN ISO 20795-2), insbesondere gemessen in Wasser bei 45 °C aufweist, und/oder
iv) a) eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C aufweist.

**13.** Rohling in Form eines dreidimensionalen Formkörpers einer polymerisierten Zusammensetzung nach einem der Ansprüche 1 bis 12 zur Herstellung dentaler prothetischer Teile, orthopädischer Apparate oder dentaler Pre-Forms, **dadurch gekennzeichnet, dass** der Rohling a.1) eine Biegefestigkeit von größer gleich 75 MPa (in Anlehnung an DIN EN ISO 20795-2) aufweist und/oder b.1) ein E-Modul von größer gleich 2000 MPa (in Anlehnung an DIN EN ISO 20795-2) aufweist und optional a.2) eine Biegefestigkeit von größer gleich 50 MPa (in Anlehnung an DIN EN ISO 20795-2), insbesondere gemessen in Wasser bei 45 °C, aufweist und/oder b.2) ein E-Modul von größer gleich 1500 MPa (in Anlehnung an DIN EN ISO 20795-2), insbesondere gemessen in Wasser bei 45 °C, aufweist.

**14.** Verwendung einer Zusammensetzung nach einem der Ansprüche 1 bis 10 zur Herstellung anatomischer Modelle, anatomischer Tischmodelle, dentaler Arbeitsmodelle, dentaler Vollmodelle, dentaler Stumpfmodelle, anatomischer oder dentaler Sägeschnittmodelle, insbesondere Situationsmodelle, Gegenbissmodelle, Funktionsmodelle, Vormodelle, Reparaturmodelle, Präzisionsmodelle, anatomischer Modelle zur Ersetzung des dentalen Gipsmodells des Gebisses, Alignern, Zahnschienen, prothetischer Teile, dentaler prothetischer Teile, orthopädischer Apparate oder dentaler Pre-Forms, insbesondere einer Zusammensetzung nach einem der Ansprüche 1 bis 11, in einem Rapid-Prototyping- oder in einem Rapid-Manufacturing- oder Rapid-Tooling-Verfahren.

**15.** Verwendung nach Anspruch 14, **dadurch gekennzeichnet, dass** die dentalen prothetischen Teile umfassen Prothesenbasis oder Teile davon, künstliche Zähne, Zahnbogen mit mindestens zwei bis 16 interdental werkstoffeinstückig verbundenen künstlichen Zähnen, Kronen, provisorische Kronen, Totalprothesen, Vollkronen, Schienen für kieferorthopädische Korrekturen (ähnlich *Invisalign*), dentale Brücken, Abutments, Suprastrukturen, dentale Stege, Inlays, Onlays, orthopädische Apparate, wie Aufbissschienen, dentale Pre-Forms von künstlichen Zähnen, Bohrschablonen für die Implantologie, Mouthguards und/oder Implantate.

Europäisches Patentamt
European Patent Office
Office européen des brevets

# EUROPÄISCHER RECHERCHENBERICHT

Nummer der Anmeldung
EP 22 16 0681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>A | US 2018/110683 A1 (YOSHINAGA KAZUHIKO [JP] ET AL) 26. April 2018 (2018-04-26)<br>* Zusammenfassung *<br>* Absätze [0001], [0307], [0774], [0779] *<br>* Tabelle 1e *<br>* Beispiel 3e * | 1,2,5, 8-15<br>3,4,6,7 | INV.<br>A61C13/00<br>A61K6/62<br>A61K6/887<br>B33Y70/00<br>B33Y80/00<br>A61C7/08<br>A61C13/01<br>A61C13/34 |
| A | IZABELA M. BARSZCZEWSKA-RYBAREK: "Characterization of urethane-dimethacrylate derivatives as alternative monomers for the restorative composite matrix", DENTAL MATERIALS, Bd. 30, Nr. 12, 18. Oktober 2014 (2014-10-18), Seiten 1336-1344, XP055698041, AMSTERDAM, NL ISSN: 0109-5641, DOI: 10.1016/j.dental.2014.09.008<br>* Zusammenfassung *<br>* Schema 1 *<br>* Tabelle 2 * | 1-15 | |
| A | US 2012/082958 A1 (BLOEMKER TOBIAS [DE] ET AL) 5. April 2012 (2012-04-05)<br>* Zusammenfassung *<br>* Absätze [0102], [0388], [0400], [0401] *<br>* Tabellen 1,2 *<br>* Beispiele 1-9 * | 1-15 | RECHERCHIERTE SACHGEBIETE (IPC)<br>A61C<br>A61K<br>B33Y |
| | -/-- | | |

Der vorliegende Recherchenbericht wurde für alle Patentansprüche erstellt

| Recherchenort | Abschlußdatum der Recherche | Prüfer |
|---|---|---|
| Den Haag | 30. Juni 2022 | Grave, Christian |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 22 16 0681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | IZABELA BARSZCZEWSKA-RYBAREK ET AL: "Comparative Study of Structure-Property Relationships in Polymer Networks Based on Bis-GMA, TEGDMA and Various Urethane-Dimethacrylates", MATERIALS, Bd. 8, Nr. 3, 19. März 2015 (2015-03-19), Seiten 1230-1248, XP055562992, CH ISSN: 1996-1944, DOI: 10.3390/ma8031230 * Zusammenfassung * * Abschnitte 2 und 3.2 * * Schema 2 * * Tabelle 2 * ----- | 1-15 | |
| A | US 2018/100075 A1 (CHOPRA NAVEEN [CA] ET AL) 12. April 2018 (2018-04-12) * Zusammenfassung * * Absätze [0061] - [0072] * * Tabelle 1A * ----- | 1-15 | RECHERCHIERTE SACHGEBIETE (IPC) |
| A | JAKUBOWSKI ET AL: "Comparison of thermomechanical properties of statistical, gradient and block copolymers of isobornyl acrylate and n-butyl acrylate with various acrylate homopolymers", POLYMER, ELSEVIER SCIENCE PUBLISHERS B.V, GB, Bd. 49, Nr. 6, 29. Januar 2008 (2008-01-29), Seiten 1567-1578, XP022518815, ISSN: 0032-3861, DOI: 10.1016/J.POLYMER.2008.01.047 * Zusammenfassung * * Seite 1568, rechte Spalte * ----- -/-- | 1-15 | |

Der vorliegende Recherchenbericht wurde für alle Patentansprüche erstellt

1

| Recherchenort | Abschlußdatum der Recherche | Prüfer |
|---|---|---|
| Den Haag | 30. Juni 2022 | Grave, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 22 16 0681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2018/055522 A1 (STRATASYS LTD [IL]) 29. März 2018 (2018-03-29)<br>* Zusammenfassung *<br>* Seite 8, Zeile 26 - Zeile 28 *<br>* Seite 46, Zeile 8 - Zeile 20 *<br>* Tabellen 1,3 *<br>----- | 1-15 | |
| A | DE 10 2015 104394 A1 (HERAEUS KULZER GMBH [DE]) 29. September 2016 (2016-09-29)<br>* Zusammenfassung *<br>----- | 1-15 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Den Haag | 30. Juni 2022 | Grave, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 22 16 0681

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-06-2022

| Im Recherchenbericht angeführtes Patentdokument | Datum der Veröffentlichung | Mitglied(er) der Patentfamilie | Datum der Veröffentlichung |
|---|---|---|---|
| US 2018110683 A1 | 26-04-2018 | CN 107427414 A | 01-12-2017 |
| | | CN 112940432 A | 11-06-2021 |
| | | EP 3278787 A1 | 07-02-2018 |
| | | JP 6634071 B2 | 22-01-2020 |
| | | JP 6826080 B2 | 03-02-2021 |
| | | JP 2018177821 A | 15-11-2018 |
| | | JP WO2016159219 A1 | 21-12-2017 |
| | | US 2018110683 A1 | 26-04-2018 |
| | | US 2020030194 A1 | 30-01-2020 |
| | | WO 2016159219 A1 | 06-10-2016 |
| US 2012082958 A1 | 05-04-2012 | EP 2436365 A2 | 04-04-2012 |
| | | US 2012082958 A1 | 05-04-2012 |
| US 2018100075 A1 | 12-04-2018 | CA 2981814 A1 | 11-04-2018 |
| | | EP 3309225 A1 | 18-04-2018 |
| | | US 2018100075 A1 | 12-04-2018 |
| WO 2018055522 A1 | 29-03-2018 | CN 109982827 A | 05-07-2019 |
| | | EP 3515687 A1 | 31-07-2019 |
| | | IL 265543 A | 31-03-2020 |
| | | IL 272959 A | 29-04-2021 |
| | | JP 6802368 B2 | 16-12-2020 |
| | | JP 2019529183 A | 17-10-2019 |
| | | KR 20190047100 A | 07-05-2019 |
| | | US 2019329488 A1 | 31-10-2019 |
| | | US 2020189179 A1 | 18-06-2020 |
| | | WO 2018055522 A1 | 29-03-2018 |
| DE 102015104394 A1 | 29-09-2016 | CN 107427347 A | 01-12-2017 |
| | | DE 102015104394 A1 | 29-09-2016 |
| | | EP 3280348 A1 | 14-02-2018 |
| | | EP 3689290 A2 | 05-08-2020 |
| | | JP 6861640 B2 | 21-04-2021 |
| | | JP 2018509248 A | 05-04-2018 |
| | | US 2018078348 A1 | 22-03-2018 |
| | | WO 2016150955 A1 | 29-09-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0130306 A, Windisch


### In der Beschreibung aufgeführte Nicht-Patentliteratur


- *Bullentin of the American Physical Society,* 1956, vol. 1 (3), 123
- **A- HOHMANN ; W. HIELSCHER.** Lehrbuch der Zahntechnik. Quintessenz Verlag, 2012, vol. 3
- **WATTS.** *Dent. Mater,* Oktober 1991, vol. 7, 281-286
- *CHEMICAL ABSTRACTS,* 4948-15-6
- *CHEMICAL ABSTRACTS,* 68186-87-8
- *CHEMICAL ABSTRACTS,* 13463-67-7
- *CHEMICAL ABSTRACTS,* 68186-90-3